# EUROPEAN PATENT SPECIFICATION

(11) **EP 3 134 081 B1**
(45) Date of publication and mention of the grant of the patent: **22.04.2020**
(21) Application number: 15828411.7
(22) Date of filing: 23.04.2015
(51) Int. Cl.: A61K 31/167, A61K 8/42, C07C 35/12, A61P 43/00, A61K 31/09, A61K 31/137, A61K 31/165, A61K 31/192, A61K 31/4402, A61K 31/485

(54) **CYCLOHEXANECARBOXAMIDE WITH COOLING PROPERTIES**
CYCLOHEXANCARBOXAMID MIT KÜHLUNGSEIGENSCHAFTEN
COMPOSITION À BASE DE CYCLOHEXANECARBOXAMIDE AVEC DES PROPRIÉTÉS DE REFROIDISSEMENT

(30) Priority: 23.04.2014 US 201461982970 P; 23.04.2014 US 201461982968 P
(43) Date of publication of application: 01.03.2017
(73) Proprietor: The Procter & Gamble Company, Cincinnati, OH 45202 (US)
(72) Inventor: HAUGHT, John, Christian, Cincinnati, Ohio 45202 (US); REILLY, Michael, Cincinnati, Ohio 45202 (US); HOKE, Steven, Hamilton, Cincinnati, Ohio 45202 (US); LEI, Qingxin, Cincinnati, Ohio 45202 (US); LIN, Yakang, Cincinnati, Ohio 45202 (US); SANKER, Lowell, Alan, Cincinnati, Ohio 45202 (US); SREEKRISHNA, Koti, Tatachar, Cincinnati, Ohio 45202 (US)
(74) Representative: Joos, Uta Susanne
(86) International application number: PCT/US2015/027249
(87) International publication number: WO 2016/036423

(56) References cited:
- WO-A1-2010/019730
- WO-A2-2007/095340
- DAVID D MCKEMY: "Therapeutic potential of TRPM8 modulators", OPEN DRUG DISCOVERY JOURNAL, BENTHAM SCIENCE PUBLISHERS B.V, NL, vol. 2, no. Special Issue 2, 1 January 2010 (2010-01-01), pages 81-88, XP008166382, ISSN: 1877-3818, DOI: 10.2174/1877381801002030081
- ECCLES R: "REVIW: MENTHOL AND RELATED COOLING COMPOUNDS", JOURNAL OF PHARMACY AND PHARMACOLOGY, JOHN WILEY & SONS LTD, LONDON; GB, vol. 46, 1 January 1994 (1994-01-01), pages 618-630, XP009076858, ISSN: 0022-3573

## Description

### FIELD OF THE INVENTION

The present invention relates to personal care compositions, such as oral care and skin care compositions, containing a flavor/perfume system comprising one or more coolants, wherein the cool sensation provided by the coolant is enhanced in terms of quicker onset, greater intensity, and/or longer duration, thereby improving appeal and acceptability of the compositions to consumers.

### BACKGROUND OF THE INVENTION

Oral care products, such as dentifrice and mouthwash, are routinely used by consumers as part of their oral care hygiene regimens. It is well known that oral care products can provide both therapeutic and cosmetic hygiene benefits to consumers. Therapeutic benefits include caries prevention which is typically delivered through the use of various fluoride salts; gingivitis prevention, by the use of an antimicrobial agent such as stannous fluoride, triclosan, essential oils; or hypersensitivity control through the use of ingredients such as strontium chloride or potassium nitrate. Cosmetic benefits provided by oral care products include the control of plaque and calculus formation, removal and prevention of tooth stain, tooth whitening, breath freshening, and overall improvements in mouth feel impression, which can be broadly characterized as mouth feel aesthetics. Calculus and plaque along with behavioral and environmental factors lead to formation of dental stains, significantly affecting the aesthetic appearance of teeth. Behavioral and environmental factors that contribute to teeth staining propensity include regular use of coffee, tea, cola or tobacco products, and also the use of certain oral products containing ingredients that promote staining, such as cationic antimicrobials and metal salts.

Thus daily oral care at home requires products with multiple ingredients working by different mechanisms to provide the complete range of therapeutic and aesthetic benefits, including anticaries, antimicrobial, antigingivitis, antiplaque, anticalculus and anti-erosion, as well as antiodor, mouth refreshment, stain removal, stain control and tooth whitening. In order for daily use oral care products, such as dentifrice and rinses to provide complete oral care it is often necessary to combine actives and additives, many of which have the disadvantage of causing negative aesthetics during use, in particular unpleasant taste and sensations and stain promotion. The unpleasant taste and mouth sensations have been described as having one or more of bitter, metallic, astringent, salty, numbing, stinging, burning, or prickling, and even irritating aspects. Typical ingredients for oral care use that are associated with these aesthetic negatives include antimicrobial agents such as cetyl pyridinium chloride, chlorhexidine, stannous and zinc salts; tooth bleaching agents such as peroxides; antitartar agents such as pyrophosphate, tripolyphosphate and hexametaphosphate; and excipients such as baking soda and surfactants. To mitigate the aesthetic negatives from these ingredients, oral care products are typically formulated with flavoring agents, sweeteners and coolants to taste as good as possible and provide a pleasant experience. In particular, it is desirable for oral care products to provide a refreshing cooling sensation during and after use. In addition to mitigation of negative sensations, sensate molecules are formulated into oral care compositions to convey a signal of efficacy. Such signals of efficacy include cooling, tingling, numbing, warming, sweetness, and rheological sensations such as phase change and fizzing or bubbling.

A large number of coolant compounds of natural or synthetic origin have been described. The most well-known compound is menthol, particularly 1-menthol, which is found naturally in peppermint oil, notably of Mentha arvensis L and Mentha viridis L. Of the menthol isomers, the 1-isomer occurs most widely in nature and is typically what is referred by the name menthol having coolant properties. L-menthol has the characteristic peppermint odor, has a clean fresh taste and exerts a cooling sensation when applied to the skin and mucosal surfaces. Other isomers of menthol (neomenthol, isomenthol and neoisomenthol) have somewhat similar, but not identical odor and taste, i.e., some having disagreeable notes described as earthy, camphor, musty. The principal difference among the isomers is in their cooling potency. L-menthol provides the most potent cooling, i.e., having the lowest cooling threshold of about 800 ppb, i.e., the concentration where the cooling effect could be clearly recognized. At this level, there is no cooling effect for the other isomers. For example, d-neomenthol is reported to have a cooling threshold of about 25,000 ppb and 1-neomenthol about 3,000 ppb. (R. Emberger and R. Hopp, "Synthesis and Sensory Characterization of Menthol Enantiomers and Their Derivatives for the Use in Nature Identical Peppermint Oils," Specialty Chemicals (1987), 7(3), 193-201). This study demonstrated the outstanding sensory properties of 1-menthol in terms of cooling and freshness and the influence of stereochemistry on the activity of these molecules.

Among synthetic coolants, many are derivatives of or are structurally related to menthol, i.e., containing the cyclohexane moiety, and derivatized with functional groups including carboxamide, ketal, ester, ether and alcohol. Examples include the p-menthanecarboxamide compounds, such as N-ethyl-p-menthan-3-carboxamide, known commercially as "WS-3", and others in the series, such as WS-5 (N-ethoxycarbonylmethyl-p-menthan-3-carboxamide), WS-12 [N-(4-methoxyphenyl)-p-menthan-3-carboxamide] and WS-14 (N-tert-butyl-p-menthan-3-carboxamide). Examples of menthane carboxy esters include WS-4 and WS-30. An example of a synthetic carboxamide coolant that is structurally unrelated to menthol is N,2,3-trimethyl-2-isopropylbutanamide, known as "WS-23". Additional examples of synthetic coolants include alcohol derivatives such as 3-(1-menthoxy)-propane-1,2-diol known as TK-10, isopulegol (under the tradename Coolact P) and p-menthane-3,8-diol (under the tradename Coolact 38D); menthone glycerol acetal known as MGA; menthyl esters such as menthyl acetate, menthyl acetoacetate, menthyl lactate known as Frescolat® supplied by Haarmann and Reimer, and monomenthyl succinate under the tradename Physcool from V. Mane. TK-10 is described in U.S. Pat. No. 4,459,425 to Amano et al. Other alcohol and ether derivatives of menthol are described e.g., in GB 1,315,626 and in U.S. Pat. Nos. 4,029,759; 5,608,119; and 6,956,139. WS-3 and other carboxamide cooling agents are described for example in U.S. Pat. Nos. 4,136,163; 4,150,052; 4,153,679; 4,157,384; 4,178,459 and 4,230,688. Additional N-substituted p-menthane carboxamides are described in WO 2005/049553A1 including N-(4-cyanomethylphenyl)-p-menthanecarboxamide, N-(4-sulfamoylphenyl)-p-menthanecarboxamide, N-(4-cyanophenyl)-p-menthanecarboxamide, N-(4-acetylphenyl)-p-menthanecarboxamide, N-(4-hydroxymethylphenyl)-p-menthanecarboxamide and N-(3-hydroxy-4-methoxyphenyl)-p-menthanecarboxamide. Other N-substituted p-menthane carboxamides include amino acid derivatives such as those disclosed in WO 2006/103401 and in US Pat. Nos. 4,136,163; 4,178,459 and 7,189,760 such as N-((5-methyl-2-(1-methylethyl)cyclohexyl)carbonyl)glycine ethyl ester and N-((5-methyl-2-(1-methylethyl)cyclohexyl)carbonyl)alanine ethyl ester. Menthyl esters including those of amino acids such as glycine and alanine are disclosed e.g., in EP 310 299 and in U.S. Pat. Nos. 3,111,127; 3,917,613; 3,991,178; 5,703,123; 5,725,865; 5,843,466; 6,365,215; 6,451,844; and 6,884,903. Ketal derivatives are described, e.g., in U.S. Pat. Nos. 5,266,592; 5,977,166 and 5,451,404. Additional agents that are structurally unrelated to menthol but have been reported to have a similar physiological cooling effect include alpha-keto enamine derivatives described in U.S. Pat. No. 6,592,884 including 3-methyl-2-(1-pyrrolidinyl)-2-cyclopenten-1-one (3-MPC), 5-methyl-2-(1-pyrrolidinyl)-2-cyclopenten-1-one (5-MPC), and 2,5-dimethyl-4-(1-pyrrolidinyl)-3(2H)-furanone (DMPF); icilin (also known as AG-3-5, chemical name 1-[2-hydroxyphenyl]-4-[2-nitrophenyl]-1,2,3,6-tetrahydropyrimidine-2-one) described in Wei et al., J. Pharm. Pharmacol. (1983), 35:110-112. Reviews on the coolant activity of menthol and synthetic coolants include H. R. Watson, et al. J. Soc. Cosmet. Chem. (1978), 29, 185-200 and R. Eccles, J. Pharm. Pharmacol., (1994), 46, 618-630.

The present invention provides compositions comprising one or more coolants, wherein the cooling and refreshing sensation provided by the coolant(s) is potentiated in terms of onset, intensity, and/or duration.

### SUMMARY OF THE INVENTION

The present invention is set out in the appended set of claims. Compounds with the following structure are described herein: 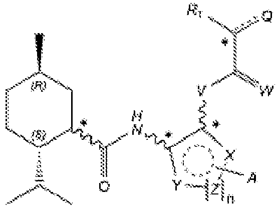
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = lower alkoxy, lower alkylthio, aryl, subsitituted aryl or fused aryl; and
stereochemistry is variable at the positions marked*.

A compound is provided having the structure shown above, wherein the compound at a concentration of about 5.2E-5% provides a greater activation of TRPM8 than WS5 at a concentration of about 30mM; a greater activation of TRPA1 than allyl isothiocyanate at a concentration of about 50mM; and a greater activation of TRPV1 than capsaicin at a concentration of about 350nM.

A compound having the structure shown above is provided, wherein the compound at a concentration of about 5.2E-5% provides at least about 100%, 105%, 110%, 115%, 120% 125% or 130% activation of TRPM8 when compared to WS5 at a concentration of about 30mM; at least about 100%, 130%, 140%, 150%, 160%, 170%, 180%, 190%, 200%, 210%, 220%, 230% or 240% activation of TRPA1 when compared to allyl isothiocyanate at a concentration of about 50mM; and at least about 95%, 100%, 105%, 110%, or 115% activation of TRPV1 when compared to capsaicin at a concentration of about 350nM.

Compounds with the following structure are described herein: 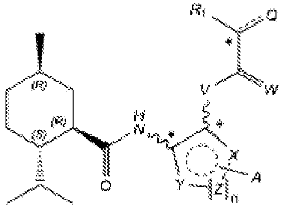
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = lower alkoxy, lower alkylthio, aryl, subsitituted aryl or fused aryl; and
stereochemistry is variable at the positions marked*.

Compounds with the following structure are described herein: 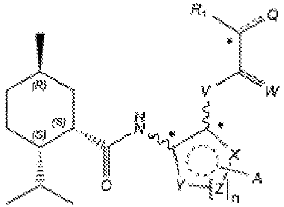
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = lower alkoxy, lower alkylthio, aryl, subsitituted aryl or fused aryl; and
stereochemistry is variable at the positions marked*.

A compound is described that comprises the following structure:

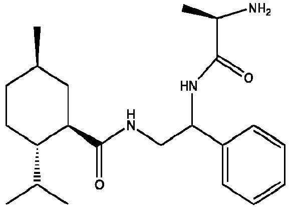

A personal care composition that comprises a compound having the following structure is described: 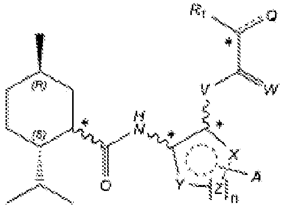
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = lower alkoxy, lower alkylthio, aryl, subsitituted aryl or fused aryl; and
stereochemistry is variable at the positions marked*; and
wherein the compound activates at least one of TRPV1, TRPV1, or TRPM8.

These and other features, aspects, and advantages of the present invention will become evident to those skilled in the art from the detailed description that follows.

### BRIEF DESCRIPTION OF THE DRAWINGS

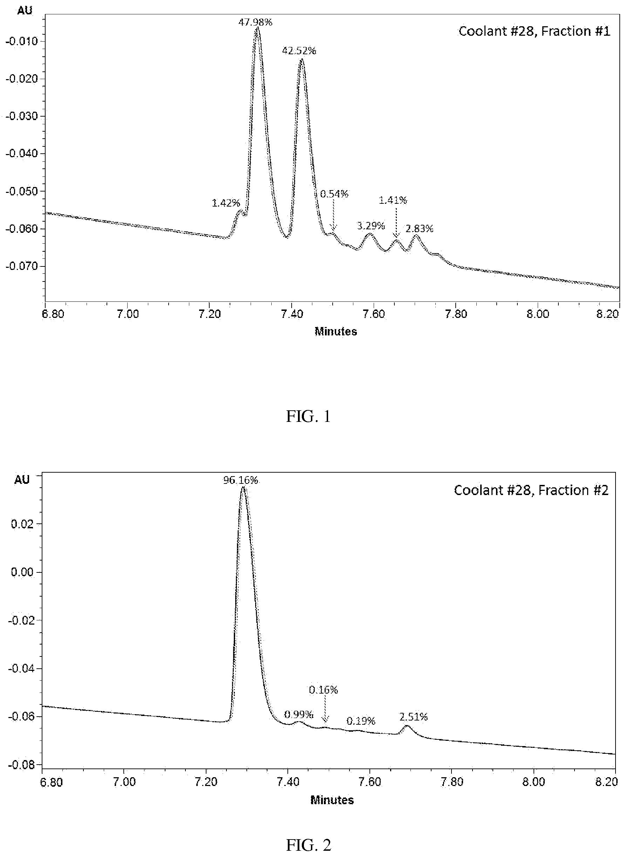

- FIG. 1: UV chromatogram overlays of three replicate injections of compound 28, fraction 1. The percentages of relative peak area are shown above each isomeric compound observed within this mixture. All peaks appear at nominal m/z 374 in the QDa mass spectra, indicating that these are isomeric species.
- FIG. 2: UV chromatogram overlays of three replicate injections of compound 28, fraction 2. The percentages of relative peak areas are shown above for each isomeric compound observed within this mixture. All peaks appear at nominal m/z 374 in the QDa mass spectra, indicating that these are isomeric species. Fraction 2 has higher isomeric purity than fraction 1.

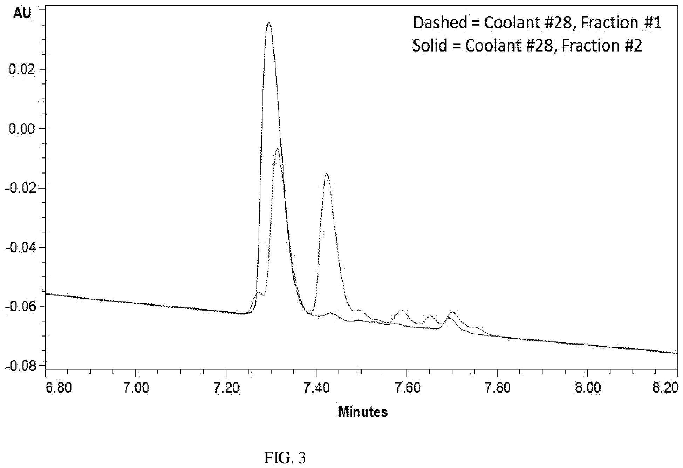

- FIG. 3: UV trace overlays of chromatograms generated during separate analysis of compound 28, fraction 1 (dashed line) and fraction 2 (solid line). These overlays demonstrate that some components are contained within both fractions, while some components are essentially unique, and the ratio of isomers within these two fractions differ. All peaks appear at nominal m/z 374 in the QDa mass spectra, indicating that these are isomeric species.

### DETAILED DESCRIPTION OF THE INVENTION

The present invention is directed to the discovery that certain cyclohexanecarboxamide structures deliver the means to drive a cooling response at low concentrations. It has been discovered that cyclohexanecarboxamide, 5-methyl-2-(1-methylethyl)-*N*-(2-phenylethyl)-, (1*R,*2*S*,5*R*) (CAS# 824947-52-6) and cyclohexanecarboxamide, 5-methyl-2-(1-methylethyl)-N-(2-phenylethyl)-, (1R,2S,5R) (CAS# 847564-71-0) structures with 2-amino-propanamide (CAS# 4726-84-5) have enhanced long lasting cooling properties and cyclohexanecarboxamide, 5-methyl-2-(1-methylethyl)-N-phenyl-, (1R,2S,5R) and cyclohexanecarboxamide, 5-methyl-2-(1-methylethyl)-N-1-naphthalenyl-(1R,2S,5R) (CAS# 863091-95-6) structures with an aminoethane (CAS# 75-04-7) moiety deliver a warming sensation. Both types of cyclohexanecarboxamide (cooling and warming) are efficacious at low use levels (1-10 ppm). The stereochemistry assigned to the compounds above is based on the dominant isomer (1R, 2S, 5R) derived from the menthol starting material. One or more additional isomers and/or enantiomers may occur due to the additional chiral sites built out from the amide linkage.

Structures built off of the cyclohexanecarboxamide backbone have been applied as anti-cancer agents as disclosed in WO 2009/067410. As shown in US Pat. No. 4,150,052, only a select few of the cyclohexanecarboxamide derivatives had noticeable cooling. The molecules disclosed in WO 2009/067410 were evaluated for their TRPM8 activity in relation to the destruction of prostate cancer cells. The data shown herein illustrates that activating TRPM8 does not necessarily mean that a cooling sensation will be observed. Thus cooling would have been an undesirable effect and something they would have avoided.

The present invention is thus based on the discovery that select molecules can be used to drive a cooling response when formulated into consumer products. A second object of this invention shows the discovery that select cyclohexanecarboxamide derivatives can provide long lasting cooling at very low levels, allowing for formulation efficiencies, in particular coolant compounds (coolants), such as described below.

All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated.

All measurements referred to herein are made at 25°C unless otherwise specified.

By "personal care composition" is meant a product, which in the ordinary course of usage is applied to or contacted with a body surface to provide a beneficial effect. Body surface includes skin, for example dermal or mucosal; body surface also includes structures associated with the body surface for example hair, teeth, or nails. Examples of personal care compositions include a product applied to a human body for improving appearance, cleansing, and odor control or general aesthetics. Non-limiting examples of personal care compositions include oral care compositions, such as, dentifrice, mouth rinse, mousse, foam, mouth spray, lozenge, chewable tablet, chewing gum, tooth whitening strips, floss and floss coatings, breath freshening dissolvable strips, denture care product, denture adhesive product; after shave gels and creams, pre-shave preparations, shaving gels, creams, or foams, moisturizers and lotions; cough and cold compositions, gels, gel caps, and throat sprays; leave-on skin lotions and creams, shampoos, body washes, body rubs, such as Vicks Vaporub; hair conditioners, hair dyeing and bleaching compositions, mousses, shower gels, bar soaps, antiperspirants, deodorants, depilatories, lipsticks, foundations, mascara, sunless tanners and sunscreen lotions; feminine care compositions, such as lotions and lotion compositions directed towards absorbent articles; baby care compositions directed towards absorbent or disposable articles; and oral cleaning compositions for animals, such as dogs and cats.

The present invention is also directed towards "oral health compositions" as used herein which refers to compositions in a form that is deliverable to a mammal in need via the oral cavity, mouth, throat, nasal passage or combinations thereof. Nonlimiting examples include liquid compositions, cough syrups, respiratory preparations, beverage, supplemental water, pills, soft gels, tablets, capsules, gel compositions, foam compositions, saline wash and combinations thereof. Liquid compositions, gel compositions can be in a form that is directly deliverable to the mouth and throat. These compositions and/or preparations can be delivered by a delivery device selected from droppers, pump, sprayers, liquid dropper, saline wash delivered via nasal passageway, cup, bottle, liquid filled gel, liquid filled gummy, center filled gum, chews, films, center filled lozenge, gum filled lozenge, pressurized sprayers, atomizers, air inhalation devices, liquid filled compressed tablet, liquid filled gelatin capsule, liquid filled capsule, squeezable sachets, power shots, and other packaging and equipment, and combinations thereof. The sprayer, atomizer, and air inhalation devices can be associated with a battery or electric power source.

The present description is also directed towards a respiratory preparation. In one embodiment the respiratory preparation comprises a film forming agent; and a thickening agent. The preparation provides on demand relief. The preparation can work to physically coat the mouth and throat creating a soothing barrier over the epithelial cells that line the throat layer. The preparation can additionally, reduce inflammation and relieve minor pain associated with a cough and/or sore throat. Preferably the respiratory preparation would not contain a pharmaceutical active.

The present invention is also directed to lotion compositions and to absorbent articles, particularly disposable absorbent articles, having a lotion treatment composition applied thereon. Disposable absorbent articles can be baby diapers or feminine hygiene articles, including incontinence devices and catamenial products, such as tampons, sanitary napkins, pantiliners, interlabial products, and the like. For convenience, the invention is disclosed below with respect to the embodiment of a catamenial device, such as a sanitary napkin or pantiliner.

The absorbent article can comprise any known or otherwise effective topsheet, such as one which is compliant, soft feeling, and non-irritating to the body of the wearer. Suitable topsheet materials include a liquid pervious material that is oriented towards and contacts the body of the wearer, thereby permitting body discharges to rapidly penetrate through the topsheet without allowing fluid to flow back through the topsheet to the skin of the wearer. The topsheet, while capable of allowing rapid transfer of fluid through it, also provides for the transfer or migration of the lotion composition onto an external or internal portion of a body of the wearer. A suitable topsheet can be made of various materials, such as woven and nonwoven materials; apertured film materials including apertured formed thermoplastic films, apertured plastic films, and fiber-entangled apertured films; hydro-formed thermoplastic films; porous foams; reticulated foams; reticulated thermoplastic films; thermoplastic scrims; or combinations thereof, as is well known in the art of making catamenial products such as sanitary napkins, pantiliners, incontinence pads, and the like.

A lotion composition of the present invention comprises at least one rheology structurant, which typically is a solid. The lotion composition can further comprise other optional ingredients, like surface energy modifiers. In one embodiment, a lotion composition consists essentially of, or consists of, a rheology structurant, such as a microcrystalline wax, alkyl dimethicone, ethylene glycol dibehenate, ethylene glycol distearate, glycerol tribehenate, glycerol tristearate, and ethylene bisoleamide. A present lotion composition can contain a single rheology structurant or a mixture of two or more rheology structurants.

In preparing a lotioned catamenial device according to the present invention, the lotion composition can be applied to the outer surface of the absorbent article, such as, for example, the outer surface of the topsheet. Any of a variety of application methods that distribute lubricious materials having a molten or liquid consistency can be used, such as, for example, as set forth in U.S. Pat. No. 5,968,025 and U.S. Pub. App. No. 2005/0208113. Suitable methods include but are not limited to spraying, printing (e.g., flexographic printing), coating (e.g., gravure coating), extrusion, dipping, or combinations of these application techniques, e.g., spraying the lotion composition on a rotating surface, such as a calender roll, that then transfers the composition to the outer surface of the sanitary napkin topsheet. Additionally, the manner of applying the lotion composition to a portion of a catamenial device can be such that the substrate or component does not become saturated with the lotion composition. The lotion composition can be applied to the catamenial device at any point during assembly. For example, the lotion composition can also be applied to the outer surface of the topsheet before it is combined with the other raw materials to form a finished catamenial device.

The term "dentifrice", as used herein, includes tooth or subgingival -paste, gel, or liquid formulations unless otherwise specified. The dentifrice composition may be a single phase composition or may be a combination of two or more separate dentifrice compositions. The dentifrice composition may be in any desired form, such as deep striped, surface striped, multilayered, having a gel surrounding a paste, or any combination thereof. Each dentifrice composition in a dentifrice comprising two or more separate dentifrice compositions may be contained in a physically separated compartment of a dispenser and dispensed side-by-side.

The term "dispenser", as used herein, means any pump, tube, or container suitable for dispensing compositions such as dentifrices.

The term "teeth", as used herein, refers to natural teeth as well as artificial teeth or dental prosthesis.

The term "orally acceptable carrier or excipients" includes safe and effective materials and conventional additives used in oral care compositions including but not limited to fluoride ion sources, anti-calculus or anti-tartar agents, buffers, abrasives such as silica, alkali metal bicarbonate salts, thickening materials, humectants, water, surfactants, titanium dioxide, flavorants, sweetening agents, xylitol, coloring agents, and mixtures thereof.

Herein, the terms "tartar" and "calculus" are used interchangeably and refer to mineralized dental plaque biofilms.

The components of the present compositions are described in the following paragraphs.

| SEQ ID NO | Sequence |
|---|---|
| 1 | Human TRPV1 DNA sequence |
| 2 | Human TRPA1 DNA sequence |
| 3 | Human TRPM8 DNA sequence |

The term "TRPV1" or "TRPV1 receptor", as used herein, refers to the transient receptor potential vanilloid receptor 1, which is a ligand-gated, non-selective cation channel preferentially expressed on small-diameter sensory neurons and detects noxious as well as other substances. The TRPV1 receptor is provided as SEQ ID NO: 1. The TRPV1 receptor responds to, for example, both noxious and painful stimuli. A noxious stimulus would include those that give a burning (i.e. hot) sensation.

The term "TRPV1 agonist", as used herein, refers to any compound, which at a concentration of 1 mM gives a calcium flux count of at least 1000 counts or 20% above the background level of calcium present in the cell according to the FLIPR method, as discussed herein. The term "count" is defined as the change in fluorescence of the cell lines due to the influx of calcium across the cell membrane, which reacts with the calcium sensitive dye present within the cells.

The term "TRPV1 antagonist", as used herein, refers to any compound which at a concentration of 1 mM gives a reduction in calcium flux count of at least 1000 counts or 20% below the activation of TRPV1 receptor by 350 µM capsaicin.

The term "TRPV1 desensitizer", as used herein, refers to any compound, which shows agonist activity and causes a decrease in activation by a known TRPV1 agonist.

The term "TRPA1" or "TRPA1 receptor", as used herein, refers to the transient receptor potential cation channel, subfamily A, member 1, having a large cysteine-rich N-terminus that contains 18 predicted ankyrin repeats. The TRPA1 receptor is provided as SEQ ID NO: 2. TRPA1 is a ligand-gated, non-selective cation channel preferentially expressed on small diameter sensory neurons.

The term "TRPA1 agonist", as used herein, refers to any compound, which at a concentration of 1 mM gives a calcium flux count of at least 1000 counts or 20% above the background level of calcium present in the cell according to the FLIPR method, as discussed herein. The term "count" is defined as the change in fluorescence of the cell lines due to the influx of calcium across the cell membrane, which reacts with the calcium sensitive dye present within the cells.

The term "TRPA1 antagonist", as used herein, refers to any compound, which at a concentration of 1 mM gives a reduction in calcium flux count of at least 1000 counts or 20% below the activation of TRPA1 receptor by 50 mM allyl isothiocyanate.

The term "TRPA1 desensitizer", as used herein, refers to any compound, which shows agonist activity and causes a decrease in activation by a known TRPA1 agonist.

The term "TRPM8" or "TRPM8 receptor", as used herein, refers to cold- and menthol-sensitive receptor (CMR1) or TRPM8. The TRPM8 nomenclature for the receptor comes from its characterization as a non-selective cation channel of the transient receptor potential (TRP) family that is activated by stimuli including low temperatures, menthol and other chemical coolants. The TRPM8 receptor is provided as SEQ ID NO: 3.

The cooling receptor conventionally known as TRPM8 or the menthol receptor has been demonstrated as a means to differentiate intensity and duration of organic molecules that initiate and propagate the non-thermal cooling perception (D.D.Mckemy, The Open Drug Discovery Journal 2:81-88 2010). McKemy reported the EC50 values of many agonists to TRPM8 which span the range of 100 nM to 19 mM, thus showing the channel can be activated across a wide range of structures at varying concentrations. This channel also has the nomenclature of CRM1 and TRPP8. The later was designated as such due to its identification with prostate cells, where it was employed as a means to identify molecules targeted towards prostate cancer.

The term "TRPM8 agonist", as used herein, refers to any compound, which when added to a TRPM8 receptor, according to the FLIPR method, as discussed herein, produces any increase in fluorescence over background.

The term "TRPM8 antagonist", as used herein, refers to any compound, which does not show any agonistic activity when directly added and inhibits activation of the TRPM8 receptor by a known TRPM8 agonist. Using the FLIPR method, as discussed herein a molecule that has >20% reduction in calcium flux compared to the WS5 activated TRPM8 receptor is considered a TRPM8 antagonist.

The term potency, as defined by the Merck Manual, refers to the concentration (EC50) or dose (ED50) of a chemistry required to produce 50% of the chemistry's maximal effect as depicted by a graded dose-response curve. EC50 equals Kd (Dissociation constant, which is a measure of 50% of the substance in question bound to the receptor) when there is a linear relationship between occupancy and response. Often, signal amplification occurs between receptor occupancy and response, which results in the EC50 for response being much less (ie, positioned to the left on the abscissa of the log dose-response curve) than KD for receptor occupancy. Potency depends on both the affinity of chemistry for its receptor, and the efficiency with which chemistry-receptor interaction is coupled to response. The dose of chemistry required to produce an effect is inversely related to potency. In general, low potency is important only if it results in a need to administer the chemistry in large doses that are impractical. Quantal dose-response curves provide information on the potency of chemistry that is different from the information derived from graded dose-response curves. In a quantal dose-response relationship, the ED50 is the dose at which 50% of individuals exhibit the specified quantal effect.

Coolants or compounds that have a physiological cooling effect particularly on oral and other mucosal surfaces and skin are common ingredients in a wide variety of products, including edible compositions, personal care compositions, and in flavor or perfume compositions. Examples of edible compositions include confectionery, candies, chocolate, chewing gum, beverages and oral medicines. Personal care compositions, including oral care compositions, have been described previously. The pleasant cooling sensation provided by coolants contributes to the appeal and acceptability of the products. In particular, oral care products, such as dentifrices and mouthwashes are formulated with coolants because they provide breath freshening effects and a clean, cool, fresh feeling in the mouth.

It is now well established that sensations such as cool or cold can be attributed to activation of receptors at peripheral nerve fibers by a stimulus such as low temperature or a chemical coolant, which produces electrochemical signals that travel to the brain, which then interprets, organizes and integrates the incoming signals into a perception or sensation. Different classes of receptors have been implicated in sensing cold temperatures or chemical coolant stimuli at mammalian sensory nerve fibers. Among these receptors, a major candidate involved in sensing cold has been identified and designated as cold- and menthol-sensitive receptor (CMR1) or TRPM8. The TRPM8 nomenclature for the receptor comes from its characterization as a non-selective cation channel of the transient receptor potential (TRP) family, which is activated by stimuli including low temperatures, menthol and other chemical coolants. However, the precise mechanisms underlying the perception of a pleasant cooling sensation on skin or oral surfaces are presently not clearly understood. While it has been demonstrated that the TRPM8 receptor is activated by menthol and other coolants, it is not fully understood what other receptors may be involved, and to what extent these receptors need to be stimulated or perhaps suppressed in order for the overall perceived sensation to be pleasant, cooling and refreshing. For example, menthol is widely used as a cooling agent, but menthol can also produce other sensations including tingling, burning, prickling and stinging as well as a minty smell and bitter taste. Thus, it can be inferred that menthol acts on many different receptors, including cold, warm, pain and taste receptors.

Examples of solvents that can be used to solubilize compounds of the present invention, such as compound 28 -as discussed below, are based upon solubility parameters and cohesion properties explained by Charles Hansen in "Hansen Solubility Parameters: A User's Handbook" by Charles M. Hansen, CRC Press (2007) and in "The CRC Handbook and Solubility Parameters and Cohesion Parameters," Edited by Allan F. M. Barton (1999). Each material is defined by three points in 3D space and these three points are known as the Hansen Solubility Parameters (HSP) which may be defined as follows.

Solubility parameters are theoretically calculated numerical constants, which are a useful tool in predicting the ability of a solvent material to dissolve a particular solute. When the solubility parameters of a solvent falls within the solubility parameter range of a solute, i.e., the material to be dissolved, solubilization of the solute is likely to occur. There are three Hansen empirically and theoretically derived solubility parameters, a dispersion-force component (δ_{D}), a polar or dipole interaction component (δ_{P}) and a hydrogen-bonding component (δ_{H}). Each of the three parameters (i.e., dispersion, polar and hydrogen bonding) represents a different characteristic of solvency, or solvent capability. In combination, the three parameters are a measure of the overall strength and selectivity of a solvent. The Total Hansen solubility parameter, which is the square root of the sum of the squares of the three parameters mentioned previously, provides a more general description of the solvency of the solvents. Individual and total Solubility Parameter units are given in MPa^{0.5}. Solubility parameters for a material may then be plotted in a normal three-dimensional graph. From the location (δ_{D}, δ_{P}, δ_{H}), a radius is projected to form a sphere, which encompasses a region of solubility such that any solvent whose parameters reside within this space should dissolve the solute in question. The distance between the HSP coordinate of material (i.e., the solute) to the HSP coordinates of material (solvent) is designated herein as Ra. The 3D distance, Ra, is defined by the equation: Ra²=4(δ_{D1}-δ_{D2})²+(δ_{P1}-δ_{P2})² +(δ_{H1}-δ_{H2})² The sphere equation of Hansen was calculated to center the target molecules of choice, in this case, compound 28 and the various isomers (L, D, and neo) and enantiomers of each. The target Polar, Dispersive, and Hydrogen Bonding HSP are the Hansen solubility parameters of the target molecule as calculated by "Molecular Modeling Pro" software, version 5.1.9 (ChemSW, Fairfield Calif., www.chemsw.com) or Hansen Solubility from Dynacomp Software. The solubility parameters of every solvent in this analysis were also calculated via this software. Within the sphere having a radius Rₐ=14 are solvents into which compound 28 and isomer materials will be soluble. For solubility >5% in the selected solvents, the preferred range of δ_{dispersion} is ±3 units, from about 15.2 to 21.2 (MPa)^{0.5}. The preferred range of δ_{polarity} is ±6 units, from about 0 to 10.8 (MPa)^{0.5}. The preferred range of δ_{Hydrogen} bonding is ±13 units, from about 0 to 25 (MPa)^{0.5}. The HSP of compound 28 were calculated as dispersion=17.8, polarity=5.6, and hydrogen bonding=9.0. Non-limiting examples of flavor and fragrance raw materials having suitable Hansen Solubility Parameters used to solubilize the carboxamide derivative include menthone, carvone, pine oil, cinnamic aldehyde, ethanol, benzyl alcohol, eucalyptol, 1,2-propane diol, 1,3-propane diol, hexane, ethanolamine, cyclodextrins, and triacetin.

Ideally, a coolant can produce a cooling or freshness sensation similar to that produced by menthol, but without certain of the disadvantages associated with menthol, such as flavor modification, bitter aftertaste, off-flavor, strong odor and burning or irritating sensation, particularly at high concentrations. It is desirable that the coolant compounds barely possess a distinctive odor or flavor while providing a pleasant fresh cool sensation of prolonged duration, in order that the effect can still be perceived for a considerable time after use, for example, longer than 15 minutes. Menthol generally provides an initial high cooling impact, but its effect is somewhat transient in that the cool sensation drops sharply within a few minutes after use. By contrast, a number of longer lasting coolant compounds may fail to provide an immediate cooling perception, i.e., within a few seconds of application, particularly when used at low levels. Thus there is a continuing need for means to potentiate the activity of coolant chemicals, in terms of quickening the onset of the cooling sensation, intensifying the cooling sensation, especially at lower concentrations, and producing a longer lasting sensation of cooling and freshness than what menthol provides.

As stated previously, the present invention is directed to the discovery that specific 5-methyl-2-(1-methylethyl)-*N*-(2-phenylethyl)-, (1*R*, 2*S*, 5*R*) cyclohexanecarboxamide structures, as shown below, deliver the means to drive a cooling response at low concentrations.

Structure I, which includes compounds of the present invention, as shown below, and which includes compound 28, represents a genus that has been surprisingly found to be useful as modulators of TRPM8 activation. Structure I represents a heteroalkyl substituted aryl or heteroalkyl-aryl substituted alkyl carboxamide of methanol having the shown below structure and including any acceptable salts or solvates thereof; wherein: 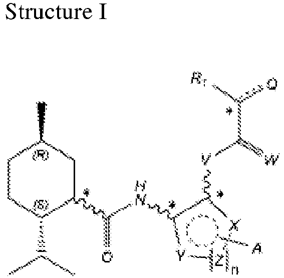
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = lower alkoxy, lower alkylthio, aryl, subsitituted aryl or fused aryl; and
stereochemistry is variable at the positions marked*.

A number of stereoisomers are contemplated in the above Structure I, where substitution is allowed and the relative configuration of each stereo center will dictate the activity towards the receptor. While it is known that the stereochemistry of side chain groups may be important to the activity of the molecule, the activity of these compounds in vivo is highly unpredictable. In some cases, isomers of the same molecule may have comparable activity. In other cases, stereoisomers of the same molecule could have enhanced or diminished activity towards the receptor. In some cases, individual stereoisomers may have no activity.

Specific compounds of interest may derive from the 1R, 2S, 5R configuration found in natural (-)-menthol. In these cases, the stereoisomeric derivatives of 1R, 2S, 5R-menthyl carboxamide will be found in the substituted alkyl side chain fragment of the molecule. While the 1R, 2S, 5R configuration is known to be important to activity, the 1S,2S,5R neo-isomer of N-substituted menthyl carboxamide derivatives has also shown promise. 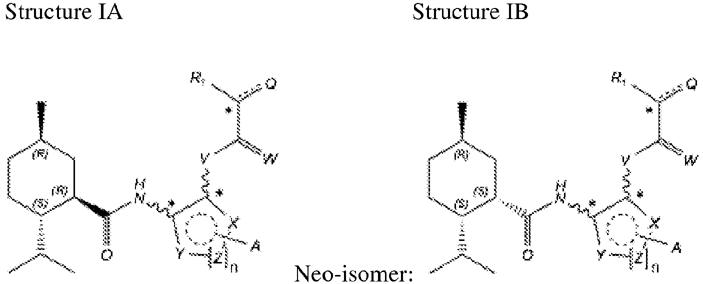
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = lower alkoxy, lower alkylthio, aryl, subsitituted aryl or fused aryl; and
stereochemistry is variable at the positions marked*.

In the case of compounds 28 and 776 (which would fall under Structure IA; and are discussed in more detail below), excellent activity is seen where the amino acid derived side chain (alanine) contains both the R (28) and S (776) configurations. In these cases, while not being limited to theory, specific activity among isomers is determined by the unique structural elements within the molecule in addition to the exact stereochemistry. While it is known that molecules having the right balance of hydrogen bonding groups (i.e. -NHR, -OH, -CONHR, etc.), Log P value, and molecular weight range are preferred, unique structural elements can contribute to activity within these preferred ranges. The current compounds of interest contain polar groups in the side-chain which are capable of both hydrogen bonding and balancing the lipophilicity of the overall structure. The stereochemical features within these molecules also impart a 3D dimensionality to the structure which can enhance interaction with specific receptors. It is believed that these unique structural features lead to enhanced affinity for the receptor which translates into the prolonged cooling effects which have been observed.

It has been discovered that cyclohexanecarboxamide, 5-methyl-2-(1-methylethyl)-*N*-(2-phenylethyl)-, (1*R*,2*S*,5*R*) (CAS# 824947-52-6) and cyclohexanecarboxamide, 5-methyl-2-(1-methylethyl)-N-(2-phenylethyl)-, (1R,2S,5R) (CAS# 847564-71-0) structures (shown above) with 2-amino-propanamide (CAS# 4726-84-5) have enhanced long lasting cooling properties and cyclohexanecarboxamide, 5-methyl-2-(1-methylethyl)-*N*-phenyl-, (1*R*,2*S*,5*R*) and cyclohexanecarboxamide, 5-methyl-2-(1-methylethyl)-N-1-naphthalenyl-(1*R*,2*S*,5*R*) (CAS# 863091-95-6) structures with an aminoethane (CAS# 75-04-7) moiety deliver a warming sensation. Both types of cyclohexanecarboxamide (cooling and warming) are efficacious at low use levels (1-10 ppm). The advantage of using such low levels of these materials allows for their formulation into higher water compositions, such as mouthrinses, without the need for additional processing aids, such as co-surfactants, oils, or other suspension agents. These materials may also provide mitigation of off tasting sensations, such as that derived from metal salts, peroxide, and CPC.

Other suitable uses for long lasting TRPM8 activity as exemplified from compound 28, would be for food applications; skin conditions, such as treatments for non-keratinzed stratified epithelium; analgesic applications as pain mitigation agents; reductions in inflammation; additives to cigarettes; topical salves for muscle pain, for chronic pain from osteoarthritis, and for chemotherapy induced neuropathy; skin barrier recovery accelerants; and antipruritic or antiseptic medications; and for vasoconstriction in relaxed vessels.

The levels of use for compounds of the present invention, such as compound 28, depend upon the targeted TRPM8 area of the body. For example in an oral application of a compound of the present invention, such as dentifrice, floss, chewing gum, or white strip, the levels of use may be from about 0.00001% to about 0.1%; from about 0.00005% to about 0.1%; from about 0.0001% to about 0.05%; or from about 0.001% to about 0.01% by weight of the composition. When a compound of the present invention is used in a mouthwash, the level of use may be from about 0.000001% to about 0.01% or from about 0.0001% to about 0.001% by weight of the composition. When a compound of the present invention, such as compound 28, is delivered topically, for example in shampoos and lotions the levels may be from about 0.001% to about 0.5% by weight of the composition or from about .01% to about 0.4% by weight of the composition.

### EXAMPLES

### EXAMPLE 1

To determine what effect, if any, test compounds (shown in TABLE 1) had on TRPM8 (SEQ ID NO: 3), TRPA1 (SEQ ID NO: 2), and TRPV1 (SEQ ID NO: 1) activation the protocols listed below were used.

### TRPM8 Protocol-FLIPR Assay

To determine whether TRPM8 is activated, the intracellular calcium ion (Ca²⁺) level was measured from transfected cells with the TRPM8 receptor sequence (SEQ ID NO: 3). HEK-293 (human embryonic kidney) cells stably transfected with human TRPM8 were grown in 15 ml growth medium (high glucose DMEM (Dulbecco's Modification of Eagle's Medium) supplemented with 10% FBS (fetal bovine serum), 100ug/ml penicillin/streptomycin, 5 µg/ml blasticindin, and 100 µg/ml zeocin) in a 75cm² flask for 3 days at 37°C in a mammalian cell culture incubator set at 5% CO₂. Cells were detached with addition of 2 ml of trypsin-EDTA buffer (GIBCO® 25200, Invitrogen, Grand Island, NY) for about 2-3 min. Trypsin was inactivated by addition of 8 ml growth medium. Cells were transferred to a 50 ml tube and centrifuged at 850 rpm for 3 minutes to remove medium. After centrifugation, a pellet of cells was formed in the bottom of the tube separating them from the supernatant solution. The supernatant was discarded and the cell pellet was suspended in 1 ml of fresh growth medium to which 5 µl (12.5 µg) of Fluo-4 AM (Molecular Probes, Inc., Eugene, OR) calcium indicator was added and incubated for 30 min with gentle shaking. Fluo-4 AM is a fluorescent dye used for quantifying cellular Ca²⁺ concentrations in the 100 nM to 1 microM range. At the end of 30 minutes, 45 ml of assay buffer (1xHBSS (Hank's Balanced Salt Solution), 20 mM HEPES (4-(2-Hydroxyethyl)-1-piperazineethanesulfonic acid)) was added to wash cells and the resulting mixture was then centrifuged at 850 rpm for 3 minutes to remove excess buffer and Fluo-4 AM calcium indicator.

The pelleted cells were re-suspended in 10 ml assay buffer and 90 µl aliquots (-50,000 cells) per well delivered to a 96-well assay plate containing 10 µl of test compounds (1 mM in assay buffer, final concentration 100 µM) or buffer control and incubated at room temperature for 30 minutes. After 30 minutes, a plate was placed into a fluorometric imaging plate reader (FLIPR384 from Molecular Devices, Sunnyvale, CA) and basal fluorescence recorded (excitation wave length 488 nm and emission wave length 510 nm). Then 20 µl of 100 mM of TRPM8 agonist WS5 coolant in the assay buffer was added and fluorescence recorded. For determining the direct effect of test compounds on TRPM8, fluorescence was measured immediately after addition of each compound (TABLES 2 and 3). Additional discussion of the FLIPR method can be found in Smart et al., Characterization using FLIPR of human vanilloid VR1 receptor pharmacology, European Journal of Pharmacology 417, 51-58 (2001) and Liu et al., Development and validation of a platelet calcium flux assay using a fluorescent imaging plate reader, Analytical Biochemistry 357, 216-224 (2006).

### TRPA1 Protocol--FLIPR Assay

To determine whether TRPA1 is activated, the intracellular calcium ion (Ca²⁺) level from transfected cells with the TRPA1 receptor sequence (SEQ ID NO: 2) was measured. HEK-293 cells stably transfected with human TRPA1 were grown in 15 ml growth medium (high glucose DMEM (Dulbecco's Modification of Eagle's Medium) supplemented with 10% FBS (fetal bovine serum), 100µg/ml penicillin/streptomycin, 100 µg/ml G418) in a 75cm² flask for 3 days at 37°C in a mammalian cell culture incubator set at 5% CO₂. Cells were detached with addition of 10 ml of PBS (phosphate buffered saline) by hand shaking gently and transferred to a 50 ml tube and centrifuged at 850 rpm for 3 minutes to remove PBS. After centrifugation, a pellet of cells was formed in the bottom of the tube separating them from the supernatant solution. The supernatant was discarded and the cell pellet suspended in 1 ml of fresh growth medium to which 5 µl (12.5 µg) of Fluo-4 AM (Molecular Probes, Inc.) calcium indicator was added and incubated for 30 minutes with gentle shaking. At the end of the 30 minutes, 45 ml of assay buffer (1xHBSS (Hank's Balanced Salt Solution), 20 mM HEPES (4-(2-Hydroxyethyl)-1-piperazineethanesulfonic acid)) was added to wash the cells and the resulting combination was then centrifuged at 850 rpm for 3 minutes to remove excess buffer and Fluo-4 AM calcium indicator.

The pelleted cells were re-suspended in 10 ml assay buffer and 90 µl aliquots (∼50,000 cells) per well delivered to a 96-well assay was placed into a fluorometric imaging plate reader (FLIPR TETRA from Molecular Devices) and basal fluorescence recorded (excitation wave length 488 nm and emission wave length 510 nm). Then 20 µl of the test compound being tested was added and fluorescence recorded (TABLES 2 and 3).

To determine if a compound was an agonist the direct effect of a test compound was determined. If any increase in fluorescence over background was noted, then the compound was considered an agonist. The agonist activity was expressed relative to that observed with a benchmark agonist such as 50 µM allyl isothiocyanate for TRPA1.

### TRPV1 Protocol--FLIPR Assay

To determine whether TRPV1 was activated, the intracellular calcium ion (Ca⁺²) levels from cells transfected with the TRPV1 receptor sequence (SEQ ID NO: 1) were measured. HEK-239 cells stably transfected with human TRPV1 were grown in 15 ml growth medium (high glucose DMEM (Dulbecco's Modification of Eagle's Medium) supplemented with 10% FBS (fetal bovine serum), 100µg/ml Penicillin/streptomycin, 100 µg/ml G418) in a 75cm² flask for 3 days at 33°C in a mammalian cell culture incubator set at 5% CO₂. Cells were detached with addition of 10 ml of PBS (phosphate buffered saline) by gentle hand shaking. Cells were transferred to a 50 ml tube and centrifuged at 850 rpm for 3 minutes to remove PBS. After centrifugation, a pellet of cells formed in the bottom of the tube separating them from the supernatant solution. The supernatant was discarded and the cell pellet suspended in 1 ml of fresh growth medium to which 5 µl (12.5 µg) of Fluo-4 AM (Molecular Probes, Inc., Eugene, OR) calcium indicator was added and incubated for 30 minutes with gentle shaking. At the end of the 30 minutes, 45 ml of assay buffer (1xHBSS (Hank's Balanced Salt Solution), 20 mM HEPES (4-(2-Hydroxyethyl)-1-piperazineethanesulfonic acid)) was added to wash the cells and the resulting combination was then centrifuged at 850 rpm for 3 minutes to remove excess buffer and Fluo-4 AM calcium indicator.

The pelleted cells were re-suspended in 10 ml assay buffer and 90 µl aliquots (∼50,000 cells) per well delivered to a 96-well assay plate was placed into a fluorometric imaging plate reader (FLIPR TETRA from Molecular Devices) and basal fluorescence recorded (excitation wave length 488 nm and emission wave length 510 nm). Then 20 µl of a test compound-being tested as a TRPV1 receptor agonist was added and fluorescence recorded. The observed value with compound pretreated cells was compared with buffer control; the difference between the two indicating a measure of effect of the test compound on the agonist (TABLES 2 and 3).

If any increase in fluorescence over background was noted, then the compound was considered an agonist. The agonist activity was expressed relative to that observed with a benchmark agonist such as 350 nM Capsaicin for TRPV1.

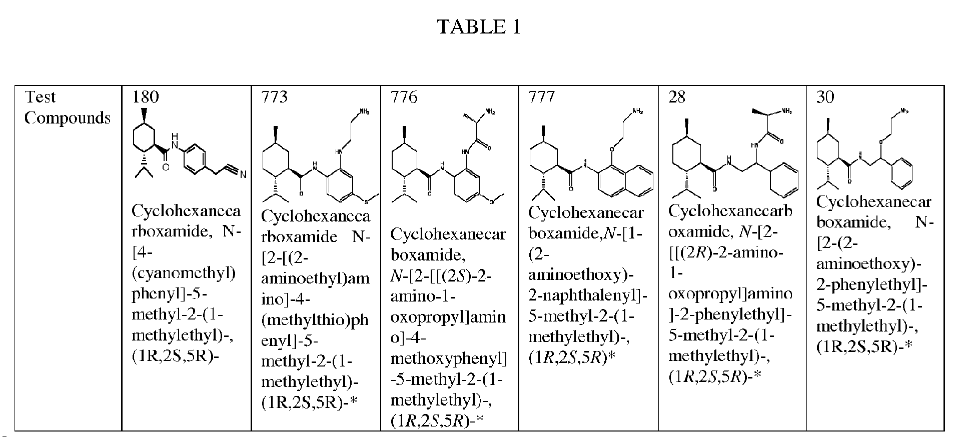

**TABLE 2: Concentration Tested on HEK 293 receptor expressing cells**

| Concentration of each test compound | TRPM8 | TRPA1 | TRPV1 |
|---|---|---|---|
| 180 | 0.001% | 0.001% | 0.001% |
| 773 | 0.004% | 0.02% | 0.02% |
| 776 | 0.004% | 0.02% | 0.02% |
| 777 | 0.004% | 0.02% | 0.02% |
| 28 | 5.2E-6% | 5.2E-5% | 5.2E-5% |
| 30 | 5.2E-5% | 5.2E-5% | 5.2E-5% |
| WS5 | 30 microMolar | - | - |
| Allyl Isothiocyanate (AITC) | - | 50 microMolar | - |
| Capsaicin | - | - | 350 nanoMolar |

TABLE 2 illustrates the concentration of each test compound when it was tested across the HEK 293 receptor containing cells.

**TABLE 3: Receptor Activity**

| Tested Compounds | TRPM8 | TRPA1 | TRPV1 |
|---|---|---|---|
| 180 | 108.80% | 68.02% | 0.01% |
| 773 | 141.07% | 198.79% | 92.07% |
| 776 | 139.41% | 112.99% | 94.61% |
| 777 | 139.37% | 173.88% | 60.5% |
| 28 | 130.42% | 242.50% | 104.50% |
| 30 | 109.70% | 38.18% | 16.24% |
| WS5 | 100% | - | - |
| Allyl Isothiocyanate (AITC) | - | 100% | - |
| Capsaicin | - | - | 100% |

TABLE 3 showed the cell based receptor activity across the three receptors (TRPM8, TRPA1, and TRPV1). Compound 28 had surprisingly high activity across all three receptors, indicating it could deliver a variety of sensations depending on the concentration, when tested in vivo.

TABLE 3 shows the impact of each structure on the following receptors: TRPM8 (cooling); TRPA1 (burning, numbing, tingling, irritation); and TRPV1 (warming). The intensity of the described test compound across three receptors (TRPM8, TRPA1, and TRPV1) was compared to that of the control test compounds (WS5 for TRPM8, Allyl Isothiocyanate for TRPA1, and Capsaicin for TRPV1). The aminoethane moiety on compounds 773 and 777 directed the underlying phenyl cyclohexanecarboxamide to shift from perceived cooling to burning (high TRPA1 activity from 777) and warming (high TRPV1 activity from 773). Further, the potency of compound 28 relative to other carboxamide structures is illustrated from TABLES 2 and 3. For instance, at a screening level of 0.0000052% compound 28 delivered 130% of WS5 activity and WS5 was tested at 0.003%. N-(4-cyanomethylphenyl)-p-menthanecarboxamide was tested at 0.001% to get to 109% of WS5 activity. Therefore, at a single concentration, compound 28 is 100X more mass efficient than the next best coolant in class, N-(4-cyanomethylphenyl)-p-menthanecarboxamide. The EC50 values, as discussed later, show it is even more mass efficient as it is diluted, since it retains high activity at very low use levels.

Compounds 28 and 30 show additional structures with the aforementioned moieties 2-amino-propanamide adjacent to a phenyl ring for cooling; and ethanolamine adjacent to a phenyl ring for warming and/or burning sensations. When the aminoethane is off a phenyl ring, the sensorial component of the molecule appeared to diminish, as shown by the lack of sensation, cooling or warming, from test compound 30. The aminoethane (test compound 777) on a naphthalene moiety delivered a burning sensation. Compound 28 was highly potent on activation of the TRPM8 receptor as shown in TABLE 3 and in the reported cooling sensations by the panelists.

### EXAMPLE 2

Sensory evaluation studies of coolant activity were conducted using a methodology patterned after the techniques described in M.C. Meilgaard, et al., Sensory Evaluation Techniques, 4th Ed. (2007). Five panelists brushed with a dentifrice for two minutes from TABLE 4 (SAMPLES A to G), SAMPLES C to G containing the test compounds in TABLE 1 in a flavor (peppermint) at 10 parts per million (ppm) and SAMPLE B containing 100 ppm of compound 180 (Cyclohexanecarboxamide, N-[4-(cyanomethyl)phenyl]-5-methyl-2-(1-methylethyl)), as the control coolant. After brush expectoration, panelists then rinsed their mouth with 15 ml of an aqueous rinse and expectorated. As shown in TABLE 5, panelists then evaluated cooling intensity, assigning a number between 0 (no cooling) to 90.

The test compounds from TABLE 1 were placed into dentifrice 4C-4G, shown in TABLE 4 and rated for their intensity and duration of cooling, as shown in TABLES 5 and 6. The scale was 0, which is no cooling sensation, to 90, which is a sensation as cold as ice.

**TABLE 4: Dentifrice formulations containing the compounds from TABLE 1**

| | SAMPLES | | | | | | |
|---|---|---|---|---|---|---|---|
| Ingredient | A (Control) | B | C | D | E | F | G |
| FD&C Blue #1 Color Solution | 0.045% | 0.045% | 0.045% | 0.045% | 0.045% | 0.045% | 0.045% |
| Sodium Fluoride | 0.243% | 0.243% | 0.243% | 0.243% | 0.243% | 0.243% | 0.243% |
| CARBOMER 956 | 0.300% | 0.300% | 0.300% | 0.300% | 0.300% | 0.300% | 0.300% |
| Sodium Saccharin | 0.300% | 0.300% | 0.300% | 0.300% | 0.300% | 0.300% | 0.300% |
| Sodium Phosphate, Monobasic, Monohydrate | 0.419% | 0.419% | 0.419% | 0.419% | 0.419% | 0.419% | 0.419% |
| Titanium Dioxide | 0.525% | 0.525% | 0.525% | 0.525% | 0.525% | 0.525% | 0.525% |
| Carboxymethycell ulose Sodium | 0.800% | 0.800% | 0.800% | 0.800% | 0.800% | 0.800% | 0.800% |
| Peppermint Flavor | 1.000% | 1.000% | 1.000% | 1.000% | 1.000% | 1.000% | 1.000% |
| Coolant | 0% | - | - | - | - | - | - |
| 180 | - | 0.01% | - | - | - | - | - |
| Compound 773 | - | - | 0.001% | - | - | - | - |
| Compound 776 | - | - | - | 0.001 % | - | - | - |
| Compound 777 | - | - | - | - | 0.001 % | - | - |
| Compound 28 | - | - | - | - | - | 0.001% | - |
| Compound 30 | - | - | - | - | - | - | 0.001% |
| Tribasic Sodium Phosphate Dodecahydrate | 1.100% | 1.100% | 1.100% | 1.100% | 1.100% | 1.100% | 1.100% |
| Sodium Lauryl Sulfate 28% Solution | 4.000% | 4.000% | 4.000% | 4.000% | 4.000% | 4.000% | 4.000% |
| Silica, Dental Type, NF (Zeodent 119) | 15.000% | 15.000 % | 15.000 % | 15.000 % | 15.000% | 15.000% | 15.000% |
| SORBITOL SOLUTION LRS USP | 54.673% | 54.673 % | 54.673 % | 54.673 % | 54.673% | 54.673% | 54.673% |
| Water Purified, USP, PhEur, JP, JSCI | QS* | QS* | QS* | QS* | QS* | QS* | QS* |

| | | | | | | | |
|---|---|---|---|---|---|---|---|
| *QS refers to the term *quantum sufficit*, meaning as much as suffices, where the remainder of the formula hole is filled with this substance. | | | | | | | |

**TABLE 5: Panelists evaluated cooling properties**

| SAMPLE | Time | Initial 0 minutes | 15 min. | 30 min | 45 min. | 60 minutes |
|---|---|---|---|---|---|---|
| C (773) | Sensory measures | 15.0 | 12.5 | 9.2 | 9.2 | 10.8 |
| D (776) | | 24.0 | 30.0 | 24.0 | 20.0 | 19 |
| E (777) | | 20.0 | 23.8 | 13.8 | 8.8 | 7.5 |
| B (180) | (0=none, 90=maximum) | 31.0 | 31.0 | 25.0 | 13.0 | 5.0 |
| F (28) | | 37.0 | 44.0 | 44.0 | 51.0 | 61.0 |
| G (30) | | 35.0 | 28.8 | 20.0 | 13.8 | 12.5 |
| A (No Coolant) | | 27.5 | 22.5 | 11.3 | 6.3 | 1.3 |

**TABLE 6: Panelists Sensory Observations**

| SAMPLE | Panel (n=5) |
|---|---|
| B (180) | 5 min delay in sensation |
| | Cool initially, then moves into tingle/burn |
| | Sensation lasts for 3 h (concentration dependent) |
| C (773) | 5 min delay in sensation |
| | Persistent warming sensation |
| | Lasts for 1 h |
| D (776) | 5 min delay in sensation |
| | Initial tingle/warm, which turned into a cooling sensation that moved to the back of the throat |
| | Lasts for 2-3 h |
| E (777) | 5 min delay in sensation |
| | Burning/tingle, almost hot sensation |
| | Lasts for 30 min. |
| F (28) | Cooling kicks in after 15 min. and lasts over 3 hours. The sensation starts on the lips and front of the mouth and progresses to the back of the throat. |
| G (30) | No cooling or other sensations observed |

Results shown in TABLES 5 and 6 showed that compound 28, shown in TABLE 1, delivered intense long-lasting cooling even at a low concentration of 10 parts per million, where compound 180 at 100 parts per million was less intense over 60 minutes. Compound 28 provided a cooling sensation from 15 minutes through the duration of the test. Most panelists commented on the sensation lasting for more than 3 hours. For compound 28, the intensity of the cooling was amplified with the drinking of water, as panelists noticed that it provided a burst of cooling sensation after the water had flowed over the soft tissues of the gum and throat. Compound 776 also displayed intense cooling that lasted for two to three hours, even though it was not as intense as compound 28. Since both compounds (776 and 28) were tested at 10 parts per million, their perceived intensity would be expected to be higher and last longer as the concentration is increased. Surprisingly, compounds 773 and 777 displayed a high degree of burning and/or warming, even though their TRPM8 activity was greater than the comparative control coolant molecule WS5. Compound 30 had no sensations, even though it had TRPM8 activity greater than WS5.

An observation of the panelists was that certain flavor types would bring out a burning/tingle sensation prior to the cooling sensation. For instance, compound 28 was combined with a Wintergreen flavor, it would display a throat burn/tingle for the first half hour of use, in addition to the cool sensation. When combined with a Peppermint flavor, it would be noted as predominately cooling. These observations were consistent with an amplifying effect of specific TRP receptors. The Wintergreen flavor inherently contains two strong TRPA1 activators in Cinnamic Aldehyde and Methyl Salicylate. Since compound 28 also has a potent TRPA1 activation in addition to TRPM8, the additional A1 signals would likely be synergistic in building a stronger A1 signal to the brain, than without the flavor A1 components. Whereas, Peppermint contains predominantly TRPM8 agonists, though with much lower TRPM8 EC50's than compound 28, and thus would be synergistic with a cooling signal. Further, addition of calcium channel enhancers (calcium soluble chelants), as exemplified in US Pub. No. 2010/0086498, may provide an additional boost to the perceived cooling by amplifying the TRPM8 sensation. Such non-limiting examples of coolant enhancers would be phytic acid, polyphosphates with a chain length of greater than or equal to 3, carboxylate polymers, such as Gantrez S-97, and polyols.

Cooling can be further enhanced by combining with select TRPV1 warming agents. Non-limiting examples of TRPV1 warming agents would be capsaicin, vanillyl butyl ether, vanillyl ethyl ether, zingerone, and piperine. Other warming agents have previously been described in US Pat. No. 6,673,844.

Combinations of compound 28 with other TRPM8 coolants may provide a quicker onset of cooling with a higher intensity than either used alone. Combining compound 28 with another coolant would allow for even less of compound 28 to be used while still providing considerable (>3 hours) freshness longevity, which may be perceived as a cooling sensation.. Examples of coolant combinations that could be used include WS23, menthane diols, menthyl carboxamide derivatives, such as WS3, WS5, N-(4-cyanomethylphenyl)-p-menthanecarboxamide, and WS12.

### EXAMPLE 3 - Isomer Characterization of compound 28

Two fractions of compound 28, as discussed below, were collected in gram quantities. The isomeric content of these two compound 28 fractions was characterized by LC-UV-MS using a Waters Acuity H Class, Ultra Performance Liquid Chromatograph (UPLC), equipped with the Sample Manager, Quaternary Solvent Manager, Tunable Ultraviolet (TUV) detector, and a QDa mass selective, single-quadrupole mass analyzer (Waters Corporation, Milford, MA). To prepare for analysis and characterization, a solid sample of each fraction was weighed and dissolved at approximately 100 µg / mL in a solution consisting of 50% deionized water / 50% methanol (MeOH, HPLC grade from EMD Millipore Corporation, Billerica, MA) and also containing 0.1% trifluoroacetic acid (TFA, Sigma Aldrich Corporation, St. Louis, MO).

The separation of isomers contained within each fraction of compound 28 was achieved with a 2.1 x 100 mm Acuity UPLC BEH Shield RP18 column with 1.7 µm particles (Waters Corporation, Milford, MA). A mobile phase gradient was utilized with mobile phase (A) consisting of water plus 0.1% TFA from Sigma Aldrich, and mobile phase (B) consisting of MeOH from EMD. The mobile phase composition was equilibrated prior to injection at 75% (A) / 25% (B) and, following a 5 µl sample injection, the mobile phase composition was ramped linearly to 100% (B) at 10 minutes. 100% of mobile phase (B) was held for 3 minutes before ramping back to the original conditions in 2 minutes. A mobile phase flow rate of 0.4 mL / minute was maintained throughout. UV traces were obtained by monitoring detector absorbance at 215 nm. QDa positive ion mass spectra of the peaks in the UV traces shown within FIG's 1 to 3 displayed intense protonated molecular ions at m/z 374, as expected, given the structure of compound 28, and indicating the components highlighted within FIG's 1 to 3 are isomeric species of compound 28.

UV analysis of compound 28 -fractions 1 and 2 are shown in FIG's 1 and 2, respectively, indicating excellent retention time repeatability and a very good separation of the isomers found within these mixtures. FIG. 3 provides a UV overlay of a representative analysis from fraction 1 and fraction 2, highlighting the differences in isomeric composition for these two fractions of compound 28.

FIG. 3 shows the HPLC of the isomers of compound 28. The fraction labeled fraction 1, collected at 7.4 to 7.5 minutes corresponds to the main isomer and lesser isomers that deliver the intense cooling and a low EC50 as determined from the TRPM8 activity as shown in TABLES 7, 8 and 9 below. The fraction labeled fraction 2, collected from 7.20 to 7.38 minutes corresponded to the isomers of compound 28 with much lower TRPM8 values, which did not provide a cooling response at the dose tested as shown in TABLES 7, 8 and 9.

TRPM8 activation was determined by measuring intracellular calcium ion (Ca²⁺) level from transfected cells with the TRPM8 receptor gene, as described in EXAMPLE 1, the results of which are shown in TABLES 7 and 8.

**TABLE 7: TRPM8 Time Course Activity of compound 28**

| Sample | Dose | 50sec | 50 sec % of WS5 | 3min | 3 min % of WS5 | 5min | 5 min % of WS5 | 10min | 10 min % of WS5 |
|---|---|---|---|---|---|---|---|---|---|
| Assay Buffer | na | 0.0 | 0.0 | 0.0 | 0.0 | 0.0 | 0.0 | 0.0 | 0.0 |
| WS-5 | 30uM | 10059.7 | 100.0 | 9449.3 | 100.0 | 9468.0 | 100.0 | 9576.0 | 100.0 |
| Compound 28 fraction 1 | 100 uM | 12646.0 | 125.7 | 12520.0 | 132.5 | 12844.0 | 135.7 | 13187.0 | 137.7 |
| | 50 uM | 12419.0 | 123.5 | 12295.0 | 130.1 | 12654.0 | 133.7 | 13169.0 | 137.5 |
| | 25 uM | 13046.0 | 129.7 | 13020.0 | 137.8 | 13354.0 | 141.0 | 14341.0 | 149.8 |
| | 12.5 uM | 12430.0 | 123.6 | 12591.0 | 133.3 | 12997.0 | 137.3 | 13947.0 | 145.6 |
| | 6.25 uM | 12229.0 | 121.6 | 12775.0 | 135.2 | 13098.0 | 138.3 | 14102.0 | 147.3 |
| | 3.125 uM | 11637.0 | 115.7 | 12602.0 | 133.4 | 12939.0 | 136.7 | 13850.0 | 144.6 |
| | 1.563 uM | 11114.0 | 110.5 | 12135.0 | 128.4 | 12499.0 | 132.0 | 13440.0 | 140.4 |
| | 781 nM | 9786.0 | 97.3 | 12182.0 | 128.9 | 12618.0 | 133.3 | 13661.0 | 142.7 |
| | 390 nM | 7592.0 | 75.5 | 11373.0 | 120.4 | 11968.0 | 126.4 | 13121.0 | 137.0 |
| | 195 nM | 5418.0 | 53.9 | 11037.0 | 116.8 | 11824.0 | 124.9 | 13046.0 | 136.2 |
| | 97.6 nM | 3963.0 | 39.4 | 9744.0 | 103.1 | 10711.0 | 113.1 | 12011.0 | 125.4 |
| | 48.8 nM | 2916.0 | 29.0 | 8017.0 | 84.8 | 8983.0 | 94.9 | 10224.0 | 106.8 |
| | 24.4 nM | 1936.0 | 19.2 | 6405.0 | 67.8 | 7593.0 | 80.2 | 8879.0 | 92.7 |
| | 12.2 nM | 3018.0 | 30.0 | 8783.0 | 93.0 | 9830.0 | 103.8 | 11065.0 | 115.5 |
| | 6.1 nM | 884.0 | 8.8 | 3452.0 | 36.5 | 4426.0 | 46.7 | 5375.0 | 56.1 |

**TABLE 8: TRPM8 Time Course Activity of Isomer of compound 28**

| Sample | Dose | 50sec | 50 sec % of WS5 | 3min | 3 min % of WS5 | 5min | 5 min % of WS5 | 10min | 10 min % of WS5 |
|---|---|---|---|---|---|---|---|---|---|
| Assay Buffer | na | 0.0 | 0.0 | 0.0 | 0.0 | 0.0 | 0.0 | 0.0 | 0.0 |
| WS-5 | 30uM | 10059.7 | 100.0 | 9449.3 | 100.0 | 9468.0 | 100.0 | 9576.0 | 100.0 |
| Compound 28 fraction 2 | 100 uM | 12246.0 | 121.7 | 11826.0 | 125.2 | 12074.0 | 127.5 | 12287.0 | 128.3 |
| | 50 uM | 12648.0 | 125.7 | 12352.0 | 130.7 | 12644.0 | 133.5 | 13179.0 | 137.6 |
| | 25 uM | 12110.0 | 120.4 | 11993.0 | 126.9 | 12284.0 | 129.7 | 13140.0 | 137.2 |
| | 12.5 uM | 12415.0 | 123.4 | 12501.0 | 132.3 | 12801.0 | 135.2 | 13755.0 | 143.6 |
| | 6.25 uM | 12236.0 | 121.6 | 12644.0 | 133.8 | 12891.0 | 136.2 | 13793.0 | 144.0 |
| | 3.125 uM | 11757.0 | 116.9 | 12699.0 | 134.4 | 12937.0 | 136.6 | 13890.0 | 145.1 |
| | 1.563 uM | 11331.0 | 112.6 | 12663.0 | 134.0 | 12954.0 | 136.8 | 13892.0 | 145.1 |
| | 781 nM | 10428.0 | 103.7 | 12887.0 | 136.4 | 13091.0 | 138.3 | 14116.0 | 147.4 |
| | 390 nM | 8766.0 | 87.1 | 11955.0 | 126.5 | 12301.0 | 129.9 | 13026.0 | 136.0 |
| | 195 nM | 7287.0 | 72.4 | 11477.0 | 121.5 | 12007.0 | 126.8 | 12427.0 | 129.8 |
| | 97.6 nM | 5007.0 | 49.8 | 10101.0 | 106.9 | 10747.0 | 113.5 | 11375.0 | 118.8 |
| | 48.8 nM | 2502.0 | 24.9 | 7721.0 | 81.7 | 8488.0 | 89.6 | 9289.0 | 97.0 |
| | 24.4 nM | 2311.0 | 23.0 | 6441.0 | 68.2 | 7226.0 | 76.3 | 7848.0 | 82.0 |
| | 12.2 nM | 1814.0 | 18.0 | 5446.0 | 57.6 | 6224.0 | 65.7 | 6809.0 | 71.1 |
| | 6.1 nM | 1944.0 | 19.3 | 4350.0 | 46.0 | 4763.0 | 50.3 | 4844.0 | 50.6 |

The TRPM8 data shown in TABLES 7 and 8, where TABLE 7 corresponds to fraction 1 and TABLE 8 corresponds to fraction 2, compares the dose response of the two HPLC separations, of the isomers (fraction 1, fraction 2) of compound 28. As shown in TABLES 7 and 8, both fractions activate TRPM8 rapidly at 781 nM of each. However, fraction 1 continued to activate at lower and lower doses compared to fraction 2. Fraction 1 was 103.8% of the control at 5 minutes of activation from a 12.2 nM dose; whereas, fraction 2 at the same time point and dose was 65.7% of the control. At 10 minutes of activation, the 12.2 nM dose was 115.5% of the control for fraction 1 and 71.1% of the control for fraction 2. These differences in isomers were further illustrated in the EC50 values as shown in TABLE 9 below.

**TABLE 9: EC50 Calculation of Isomer fractions**

| EC50 in TRPM8 (µM) | 50 sec | 3 min | 5 min | 10 min |
|---|---|---|---|---|
| fraction 2 | 0.1972 | 0.05548 | 0.04567 | 0.0374 |
| fraction 1 | 0.3306 | 0.001476 | ∼ 5.235E-008 | ∼ 1.148E-007 |

In an oral application of a compound of the present invention, such as from a dentifrice, lozenge, floss, chewing gum, or white strip, when compound 28 is split into isomers or combined, the levels of use may be from about 10% to about 70% of fraction 1 and about 10% to about 70% of fraction 2 or from about 30% to about 60% of fraction 1 and about 30% to about 60% of fraction 2. When compound 28, either isomer or combined isomers, is combined with a TRPA1 agonist, TRPV1 agonist, or both, the level of use of a TRPA1 or TRPV1 agonist would be in the range of about 0.001% to about 0.5% or from about 0.01% to about 0.2% by weight of the composition of either the TRPA1 or TRPV1 agonists, where both TRPA1 agonists and/or TRPV1 agonists may be added separately or simultaneously to the composition containing compound 28. When another TRPM8 agonist, in addition to compound 28, is used, the level of use of the additional TRPM8 agonist may be from about 0.001% to about 0.5% or from about 0.005% to about 0.3% by weight of the composition. If a TRPM8 enhancer is used, in addition to compound 28, it may be added in a range of from about 0.001% to about 0.2% or from about 0.005% to about 0.1% by weight of the composition. Compositions of the present invention may contain multiple TRPA1 and TRPV1 agonists in the ranges disclosed above to deliver the enhanced sensorial signal from compound 28.

In a topical application of a compound of the present invention, for example in shampoos and lotions, when compound 28 is split into isomers or combined, the levels of use may be from about 10% to about 70% of fraction 1 and about 10% to about 70% of fraction 2 or from about 30% to about 60% of fraction 1 and about 30% to about 60% of fraction 2. When compound 28, either isomer or combined isomers, is combined with a TRPA1 and/or a TRPV1 agonist, the level of use of a TRPA1 or TRPV1 agonist may be in the range of from about 0.001% to about 0.5% or from about 0.01% to about 0.2% by weight of the composition of either of the TRPA1 or TRPV1 agonists, where both TRPA1 agonists and TRPV1 agonists may be added separately or simultaneously to the composition containing compound 28. When another TRPM8 agonist is used, in addition to compound 28, the level of use of the additional TRPM8 agonist may be from about 0.001% to about 0.5% or from about 0.005% to about 0.3% by weight of the composition. If a TRPM8 enhancer is used, in addition to compound 28, it may be used in levels of from about 0.001% to about 0.2% or from about 0.005% to about 0.1% by weight of the composition. The compositions may contain multiple TRPA1 and TRPV1 agonists in the ranges stated to deliver the enhanced sensorial signal from compound 28.

### EXAMPLE 4: Compound 28 Solubility

**TABLE 10: Solubility Parameter Calculation**

| Compound | Dispersion (MPa)^0.5 | Polarity (MPa)^0.5 | Hydrogen bonding (MPa)^0.5 | Total Solubility Parameter (MPa)^0.5 |
|---|---|---|---|---|
| Compound 28 | 17.8 | 5.6 | 9.0 | 20.7 |

TABLE 10 outlines the Hansen solubility parameters for compound 28 and its isomers (fraction 1, fraction 2), as outlined previously. These parameters help to identify which solvents would be a good candidate for making stock solutions of >5%. Due to the low level of use in oral care products, a typical stock solution of ∼1% would be sufficient to deliver 1 to 10 ppm. Solvents for the higher (>5%) would utilize Hansen's sphere calculations and a sphere radius in the range of 5-6 would sufficiently identify solvents for the higher stock solutions. In the range of oral care use of stock solutions in the 1-5% range, solvents such as ethanol, menthol, carvone, anethol, benzyl alcohol, and the polyols commonly used in oral care products could be used to make a stock solution.

### EXAMPLE 5: Mouthwash Isomer Cooling

Mouthwashes were prepared, using conventional methods, which contained either fraction 1 or fraction 2 of compound 28 and provided to panelists to assess their cooling properties. The panelists swished the mouthwash in their mouths for 1 minute prior to expectoration. After expectoration, the time point of zero was started and time after rinse was counted from there until 12 hours. The panelists then rated the perceived refreshing experience as experienced by breathing in and noting the cool/refreshing sensation on the interior of the mouth and across the lips. The self-rated scale was from zero (no cooling) to 100 (maximum cooling).

**TABLE 11: Mouthwash Formulation**

| Ingredients | Control | Sample A | Sample B | Sample C |
|---|---|---|---|---|
| Cetylpyridinium Chloride USP | 0.074% | 0.074% | 0.074% | 0.074% |
| Compound 28 fraction 1 | 0 | 0.00005% | 0 | 0.0001% |
| Compound 28 fraction 2 | 0 | 0.00005% | 0.0001% | 0 |
| Superol Vegetable 99.7% Glycerine USP/FCC | 5% | 5% | 5% | 5% |
| Poloxamer 407 | 0.06% | 0.06% | 0.06% | 0.06% |
| Sucralose NF | 0.015% | 0.015% | 0.015% | 0.015% |
| Saccharin Sodium USP Granular, High Moist | 0.01% | 0.01% | 0.01% | 0.01% |
| Methyl Paraben | 0.02% | 0.02% | 0.02% | 0.02% |
| Propyl Paraben | 0.005% | 0.005% | 0.005% | 0.005% |
| Peppermint Flavor | 0.1% | 0.1% | 0.1% | 0.1% |
| Purified Water USP (Bottled) | QS | QS | QS | QS |

| | | | | |
|---|---|---|---|---|
| *QS refers to the term *quantum sufficit*, meaning as much as suffices, where the remainder of the formula hole is filled with this substance. | | | | |

**TABLE 12: Results from panel testing of mouthwash with coolant fractions**

| | Cool burn/thermal diffusion | | | | | | | | |
|---|---|---|---|---|---|---|---|---|---|
| | Hours after use attributes | | | | | | | | |
| Rinse | 1h | 2h | 3h | 4h | 5h | 6h | 7h | 8h | 9h |
| Sample A | 53 | 63 | 60 | 47 | 43 | 40 | 40 | 37 | 27 |
| Sample B | 27 | 10 | 3 | 0 | 0 | 0 | 0 | 0 | 0 |
| Sample C | 15 | 30 | 50 | 60 | 60 | 50 | 45 | 45 | 40 |
| Control | 5 | 0 | 0 | 0 | 0 | 0 | 0 | 0 | 0 |

The results in Table 12 showed that the mixture of fraction 1 and 2 of compound 28 (Sample A) delivered higher intensity of cooling during the first two hours after use compared with either fraction 1 (Sample C) or fraction 2 (Sample B) alone. The differences in cooling between fraction 1 (Sample C) and fraction 2 (Sample B) showed that the isomer (fraction 1) of compound 28 as depicted in FIG. 1 by the peak at 7.4 minutes was the isomer (fraction 1) that provided long lasting cooling properties. The isomer (fraction 2) corresponding to the peak from 7.20 to 7.38 minutes in FIG. 2 did not provide a strong nor long lasting cooling sensation. It was described as more of a burn/tingle than cool and the sensation trailed off in intensity between 1 and 2 hours. It does appear that there was synergy between fraction 1 and 2 where the non-cooling fraction 2 lifted the intensity of fraction 1. The intensity and duration of cooling for fraction 1 was unlike any previously tested coolant in that only 1 ppm of total coolant (compound 28 -fraction 1 and 2) was able to deliver a cooling effect, either singly (fraction 1 or 2) or as a mixture of isomers (fraction 1 and 2) where even the most powerful commercially available coolant would take at least 15 ppm to deliver a similar initial intensity, but would not be able to match the duration of compound 28.

### EXAMPLE 6: Chewing Gum Cooling

### Chewing gum preparation

The chewing gum formulations shown in TABLE 13 were prepared by melting chewing gum base in a microwave in 20 second increments until softened. Magnesium stearate was spread on a piece of wax paper and the softened chewing gum base was placed on the magnesium stearate coated portion of the wax paper. The gum base was coated with the magnesium stearate and kneaded with gloved hands until soft. The gum base was pressed to 1/4 inch thick slabs; powdered sweetener and spray dried powdered flavor were added to the center of the surface of the gum. Added liquid flavor and two drops of colorant on top of the powdered sweetener and spray dried powdered flavor, letting the liquid flavor and colorant absorb into the spray dried powdered flavor and powdered sweetener, allowing mixing it into the gum base without losing any materials. Microwaved as needed to keep the gum base pliable. Sprinkled magnesium stearate and powdered sweetener (1.2 grams) onto the wax paper to be able to roll out without the gum base sticking and also to coat the outside with a small amount of sweetener. The gum base was set onto the magnesium stearate and powdered sweetener. Coated a stainless steel beaker in magnesium stearate and used it to press/roll out the outside of the gum base for initial sweetness. Used a molded press to cut the gum base into long strips and cut again in the perpendicular direction to get squares of ∼1.0 grams each. For the batch that contains the compound 28 fraction 1, the compound 28 fraction 1 was added to the liquid flavor and prepared as described above to give a finished concentration of 1 ppm.

**TABLE 13: Gum Formulation**

| Ingredients | Control (%) | Coolant Formulation |
|---|---|---|
| Compound 28 fraction 1 | 0.0 | 0.0001 |
| Spearmint Flavor Liquid | 3.992% | 3.992% |
| Spearmint spray dried flavor | 8% | 8% |
| Sucralose | 1% | 1% |
| Chewing gum base | QS to 25 grams | QS to 25 grams |
| Coating of finished gum | 1.245 g Xylitol/stearate per 1 gram cube of gum | 1.245 g Xylitol/stearate per 1 gram cube of gum |

TABLE 14 shows the results of panelists sampling chewing gum controls and chewing gum containing compound 28 fraction 1. Panelists chewed the gum for 30 minutes and rated the in use chewing attributes of the gum. Those attributes were sweetness, flavor intensity, cooling, bitterness, and perception of freshness. The panelists rated these attributes on a 0 (no sensation of the attribute) to 100 (maximum sensation of the attribute). The data was reported on a gum without the coolant (control) compared to a gum containing compound 28 fraction 1.

**TABLE 14: Chewing gum panel in use attributes**

| During Chewing Attributes | Sweetness | Flavor Intensity | Cooling | Bitterness | Perception of Freshness |
|---|---|---|---|---|---|
| Control Gum | 43.3 | 33.3 | 30.0 | 6.7 | 30.0 |
| Gum with compound 28 fraction 1 | 56.7 | 36.7 | 36.7 | 6.7 | 33.3 |

The gum containing compound 28 fraction 1 showed improvements in the in use sweetness profile, as shown in TABLE 14.

After the panelists stopped chewing the gum as described above, they continued to rate the perception of freshness of their breath and mouth, as shown in TABLE 15. The panelists monitored the perception of freshness delivered from the gum after use by breathing in and noting the cool sensation, along with the perception of taste, and the overall perceived feeling in their mouth and on their lips. They rated from 0 (no perception of freshness) to 100 (maximum perception of freshness) over the course of 4 hours, rating at each hour. The data is reported in Table 15 below.

**TABLE 15: After Chewing Attributes**

| After Use Perception of Freshness | 1h | 2h | 3h | 4h |
|---|---|---|---|---|
| Control Gum | 6.7 | 0.0 | 0.0 | 0.0 |
| Gum with compound 28 fraction 1 | 33.3 | 33.3 | 30.0 | 26.7 |

The data reported in TABLE 15 showed that the gum containing compound 28 fraction 1 delivered a long lasting perception of freshness, as compared to the control gum.

### EXAMPLE 7

TABLES 16 and 17 show shave prep compositions. The water soluble polymers (poly-ethylene oxide, hydroxyethylcellulose) were added to water and mixed by stirring until the polymers were completely dissolved (about 30 min.). The aqueous mixture was then heated and the glyceryl oleate, sorbitol and fatty acids added at about 60°C and mixed by stirring well while the heating continues. At 80-85 °C the triethanolamine was added and mixed for about 20 minutes to form the aqueous soap phase. After cooling the aqueous soap phase to room temperature (∼25°C), the remaining components (i.e., Lubrajel, glycerin, fragrance, colorant, botanicals) were added to the aqueous soap phase and mixed by stirring well to form the gel concentrate. Water was added if required to bring the batch weight to 100%, thereby compensating for any water loss due to evaporation. The concentrate was then combined with the volatile post-foaming agent under pressure within the filling line and filled into bottom-gassed aerosol cans with shearing through the valve under nitrogen pressure.

**TABLE 16: Shave Prep Compositions**

| | Samples | | | |
|---|---|---|---|---|
| Ingredients | 1 | 2 | 3 | 4 |
| Sorbitol 70% Solution | 0.97% | 0.97% | 0.97% | 0.97% |
| Glycerin | 0.49% | 0.49% | 0.49% | 0.49% |
| Water | QS | QS | QS | QS |
| Hydroxyethyl cellulose¹ | 0.49% | 0.49% | 0.49% | 0.49% |
| PEG-90M² | 0.06% | 0.06% | 0.06% | 0.06% |
| PEG-23M³ | 0.05% | 0.05% | 0.05% | 0.05% |
| PTFE | 0.15% | 0.15% | 0.15% | 0.15% |
| Palmitic acid | 7.53% | 7.53% | 7.53% | 7.53% |
| Stearic acid | 2.53% | 2.53% | 2.53% | 2.53% |
| Glyceryl Oleate | 1.94% | 1.94% | 1.94% | 1.94% |
| Triethanolamine (99%) | 5.88% | 5.88% | 5.88% | 5.88% |
| Lubrajel Oil⁴ | 0.4% | 0.4% | 0.4% | 0.4% |
| Menthol | 0.15% | 0.15% | 0.15% | 0.15% |
| Fragrance | 0.87% | 0.87% | 0.87% | 0.87% |
| Other (e.g. Vit E, Aloe, etc.) | 0.10% | 0.10% | 0.10% | 0.10% |
| Compound 776 | - | 0.0001% | - | - |
| Compound 28 | - | - | 0.0001% | 0.1% |
| Dye | 0.10% | 0.10% | 0.10% | 0.10% |
| Isopentane (and) Isobutane | 2.85% | 2.85% | 2.85% | 2.85% |

| | | | | |
|---|---|---|---|---|
| ¹Available as Natrosol 250 HHR from Hercules Inc., Wilmington, DE ²Available as Polyox WSR-301 from Amerchol Corp., Piscataway, NJ ³Available as Polyox WSR N-1 2K from Amerchol Corp., Piscataway, NJ ⁴Available as Microslip 519 from Micro Powders Inc., Tarrytown, NY ⁴Available from Guardian Laboratories, Hauppauge, NY *QS refers to the term quantum sufficit, meaning as much as suffices, where the remainder of the formula hole is filled with this substance | | | | |

The pre-shave prep samples shown in TABLE 17 were made by weighing out the water in a vessel sufficient to hold the entire batch. Inserted an overhead mixer with impeller into the vessel and increase agitation to create a vortex. Pre-blended the thickener and polymer powders (Polyox and HEC). Sprinkled the polymer blend into the vortex until incorporated. Heated batch to 70°C to hydrate the polymers. Added the liquid dispersion polymer (e.g., Sepigel) to the batch and mixed until uniform and hydrated, increasing rpms to maintain good mixing. Added the surfactant (e.g., Brig 35) and mixed until uniform and dispersed. Cooled batch to below 45°C. Once below 45°C, added the perfume, preservatives and other temperature-sensitive additives. Cooled to below 35°C and QS with water.

**TABLE 17: Pre-Shave Prep**

| | Samples | | | |
|---|---|---|---|---|
| Ingredients | 1 | 2 | 3 | 3 |
| Water | QS | QS | QS | QS |
| Sepigel 305 (Polyacrylamide & C13-C14 Isoparaffin & Laureth-7) | 0.50% | 0.50% | 0.50% | 0.50% |
| Polyox N13K (PEG-23M) | 0.50% | 0.50% | 0.50% | 0.50% |
| Natrosol 250 HHR (HEC) | 0.80% | 0.80% | 0.80% | 0.80% |
| Glycerin 99.7% USP/Fcc | 5.0% | 5.0% | 5.0% | 5.0% |
| Brij 35 (Laureth 23) | 2.0% | 2.0% | 2.0% | 2.0% |
| Disodium EDTA | 0.10% | 0.10% | 0.10% | 0.10% |
| Perfume | 0.15% | 0.15% | 0.15% | 0.15% |
| Glydant Plus | 0.20% | 0.20% | 0.20% | 0.20% |
| Menthol | 0.04% | 0.04% | 0.04% | 0.04% |
| Compound 776 | 0.000001% | 0.000001% | 0.000001% | 0.1% |
| Compound 28 | 0.000001% | 0.000001% | 0.000001% | 0.1% |

### EXAMPLE 8

Shampoo compositions, as shown below in TABLE 18, were prepared using conventional methods.

**TABLE 18**

| | Samples | | | | | | | |
|---|---|---|---|---|---|---|---|---|
| Ingredients | A | B | C | D | E | F | G | H |
| Sodium Laureth Sulfate (SLE₃S) | | | | | | 6 | 6 | 6 |
| Sodium Laureth Sulfate (SLE₁S) | 10.5 | 10.5 | 12 | 12 | 12 | | | |
| Sodium Lauryl Sulfate (SLS) | 1.5 | 1.5 | | | | 7 | 7 | 7 |
| Cocamidopropyl Betaine | 1 | 1.25 | 1.5 | 1.5 | 1.5 | 1 | 1 | 1 |
| Cocamide MEA | 1 | 1.5 | 1.5 | 1.5 | 1.5 | | | |
| Glycol Distearate | 1.5 | 1.5 | 1.5 | 1.5 | 1.5 | 1.5 | 1.5 | 1.5 |
| Zinc Pyrithione | 1 | 1 | 1 | 1 | 1 | 1 | 1 | 1 |
| Zinc Carbonate | 1.61 | 1.61 | 1.61 | 1.61 | 1.61 | 1.61 | 1.61 | 1.61 |
| Menthol | 0.45 | 0.45 | 0.45 | 0.45 | 0.45 | 0.45 | 0.45 | |
| Compound 28 | 0.09 | 0.09 | 0.09 | 0.09 | 0.09 | 0.09 | 0.09 | 0.09 |
| Fragrance | 0.7 | 0.7 | 0.7 | 0.7 | 0.7 | 0.7 | 0.7 | 0.7 |
| Guar Hyrdroxypropyltrimonium Chloride (LMW) | 0.3 | 0.3 | 0.3 | 0.3 | 0.3 | 0.23 | 0.23 | 0.23 |
| Polyquaternium-10 (HMW) | | | 0.1 | 0.1 | 0.1 | | | |
| Polyquaternium 76 (AM:Triquat) | 0.01 | 0.01 | | | | | 0.01 | 0.01 |
| Stearyl Alcohol | | | | 1.29 | | | | |
| Cetyl Alcohol | | | 0.71 | 0.71 | | | | |
| Dimethicone | 1.7 | 0.8 | 0.8 | 0.8 | 1.7 | 0.8 | 0.8 | 0.8 |
| Hydrochloric acid | QS | QS | QS | QS | QS | QS | QS | QS |
| Preservative | 0.05 | 0.05 | 0.05 | 0.05 | 0.05 | 0.05 | 0.05 | 0.05 |
| Sodium Chloride | QS | QS | QS | QS | QS | QS | QS | QS |
| Sodium Xylene Sulfonate | QS | QS | QS | QS | QS | QS | QS | QS |
| Sodium Benzoate (22) | 0.27 | 0.27 | 0.27 | 0.27 | 0.27 | 0.27 | 0.27 | 0.27 |
| Water and Minors (QS to 100%) (23) | QS | QS | QS | QS | QS | QS | QS | QS |

Conditioner compositions, as shown below in TABLES 19-23, were prepared using conventional methods.

### Definitions of Components used in TABLES 19-22

1.Polyquaternium-6:Poly(diallyldimethylammonium chloride) supplied with a tradename Merquat 100 from Lubrizol, having a charge density of about 6.2meq/g, and molecular weight of about 150,000g/mol
2. Polyquaternium-6:Poly(diallyldimethylammonium chloride) supplied with a tradename Merquat 106 from Lubrizol having a charge density of about 6.2meq/g, and molecular weight of about 15,000g/mol
3. Zinc pyrithione: having a particle size of from about 1 to about 10 microns
4. Zinc carbonate: having a particle size of from about 1 to about 10 microns
5. Aminosilicone: Terminal aminosilicone which is available from Momentive Performance Materials having a viscosity of about 10,000mPa•s, and having following formula:

   (R₁)ₐG₃₋ₐ-Si-(-OSiG₂)ₙ-O-SiG₃₋ₐ(R₁)ₐ

   wherein G is methyl; a is an integer of 1; n is a number from 400 to about 600; R₁ is a monovalent radical conforming to the general formula C_{q}H_{2q}L, wherein q is an integer of 3 and L is -NH₂.

**TABLE 19**

| | Samples | | | | |
|---|---|---|---|---|---|
| Ingredients | 1 | 2 | 3 | 4 | 5 |
| Polyquaternium-6¹ | 0.075 | - | - | - | 0.075 |
| Polyquaternium-6² | | 0.075 | 0.075 | 0.075 | |
| Zinc pyrithione³ | 0.75 | 0.75 | 0.75 | 0.75 | 0.75 |
| Zinc carbonate⁴ | 1.6 | 1.6 | 1.6 | 1.6 | 1.6 |
| Behenyl trimethyl ammonium chloride | - | - | - | 2.5 | |
| Behenyl trimethyl ammonium methosulfate | 2.6 | 2.6 | 1.2 | - | 2 |
| Dicetyl dimethyl ammonium chloride | - | - | 0.35 | - | - |
| Cetyl alcohol | 1 | 1 | 1 | 1 | 1.4 |
| Stearyl alcohol | 2.4 | 2.4 | 2.3 | 2.3 | 3.4 |
| Aminosilicone⁵ | 0.5 | 0.5 | 0.5 | 0.5 | 2 |
| Preservatives | 0.9 | 0.9 | 0.9 | 0.9 | 0.9 |
| Compound 28 | 0 | 0 | 0 | 0 | 0 |
| Perfume | 0.5 | 0.5 | 0.5 | 0.5 | 0.5 |
| Deionized Water | q.s. to 100% | q.s. to 100% | q.s. to 100% | q.s. to 100% | q.s. to 100% |

**TABLE 20**

| | Samples | | | | |
|---|---|---|---|---|---|
| Ingredients | 6 | 7 | 8 | 9 | 10 |
| Polyquaternium-6¹ | 0.075 | - | - | - | 0.075 |
| Polyquaternium-6² | | 0.075 | 0.075 | 0.075 | |
| Zinc pyrithione ³ | 0.75 | 0.75 | 0.75 | 0.75 | 0.75 |
| Zinc carbonate⁴ | 1.6 | 1.6 | 1.6 | 1.6 | 1.6 |
| Behenyl trimethyl ammonium chloride | - | - | - | 2.5 | |
| Behenyl trimethyl ammonium methosulfate | 2.6 | 2.6 | 1.2 | - | 2 |
| Dicetyl dimethyl ammonium chloride | - | - | 0.35 | - | - |
| Cetyl alcohol | 1 | 1 | 1 | 1 | 1.4 |
| Stearyl alcohol | 2.4 | 2.4 | 2.3 | 2.3 | 3.4 |
| Aminosilicone⁵ | 0.5 | 0.5 | 0.5 | 0.5 | 2 |
| Preservatives | 0.9 | 0.9 | 0.9 | 0.9 | 0.9 |
| Compound 28 | 0.09 | 0.09 | 0.09 | 0.09 | 0.09 |
| Perfume | 0.5 | 0.5 | 0.5 | 0.5 | 0.5 |
| Deionized Water | q.s. to 100% | q.s. to 100% | q.s. to 100% | q.s. to 100% | q.s. to 100% |

**TABLE 21**

| | Samples | | | | |
|---|---|---|---|---|---|
| Ingredients | 11 | 12 | 13 | 14 | 15 |
| Polyquaternium-6¹ | 0.075 | - | - | - | 0.075 |
| Polyquaternium-6² | | 0.075 | 0.075 | 0.075 | |
| Zinc pyrithione³ | 0.75 | 0.75 | 0.75 | 0.75 | 0.75 |
| Zinc carbonate ⁴ | - | - | - | - | - |
| Behenyl trimethyl ammonium chloride | - | - | - | 2.5 | |
| Behenyl trimethyl ammonium methosulfate | 2.6 | 2.6 | 1.2 | - | 2 |
| Dicetyl dimethyl ammonium chloride | - | - | 0.35 | - | - |
| Cetyl alcohol | 1 | 1 | 1 | 1 | 1.4 |
| Stearyl alcohol | 2.4 | 2.4 | 2.3 | 2.3 | 3.4 |
| Aminosilicone⁵ | 0.5 | 0.5 | 0.5 | 0.5 | 2 |
| Preservatives | 0.9 | 0.9 | 0.9 | 0.9 | 0.9 |
| Compound 28 | 0 | 0 | 0 | 0 | 0 |
| Perfume | 0.5 | 0.5 | 0.5 | 0.5 | 0.5 |
| Deionized Water | q.s. to 100% | q.s. to 100% | q.s. to 100% | q.s. to 100% | q.s. to 100% |

**TABLE 22**

| | Samples | | | | |
|---|---|---|---|---|---|
| Ingredients | 16 | 17 | 18 | 19 | 20 |
| Polyquaternium-6¹ | 0.075 | - | - | - | 0.075 |
| Polyquaternium-6 ² | | 0.075 | 0.075 | 0.075 | |
| Zinc pyrithione³ | 0.75 | 0.75 | 0.75 | 0.75 | 0.75 |
| Zinc carbonate⁴ | - | - | - | - | - |
| Behenyl trimethyl ammonium chloride | - | - | - | 2.5 | |
| Behenyl trimethyl ammonium methosulfate | 2.6 | 2.6 | 1.2 | - | 2 |
| Dicetyl dimethyl ammonium chloride | - | - | 0.35 | - | - |
| Cetyl alcohol | 1 | 1 | 1 | 1 | 1.4 |
| Stearyl alcohol | 2.4 | 2.4 | 2.3 | 2.3 | 3.4 |
| Aminosilicone⁵ | 0.5 | 0.5 | 0.5 | 0.5 | 2 |
| Preservatives | 0.9 | 0.9 | 0.9 | 0.9 | 0.9 |
| Compound 28 | 0.09 | 0.09 | 0.09 | 0.09 | 0.09 |
| Perfume | 0.5 | 0.5 | 0.5 | 0.5 | 0.5 |
| Deionized Water | q.s. to 100% | q.s. to 100% | q.s. to 100% | q.s. to 100% | q.s. to 100% |

Samples 6-10, in TABLE 20, provided a cooling sensation to consumers in comparison to Samples 1-5, in TABLE 19. In Samples 16-20, TABLE 22, there was a cooling sensation to consumers in comparison to Samples 11-15, in TABLE 21.

**TABLE 23**

| | Samples | | | | | |
|---|---|---|---|---|---|---|
| Ingredients | 21 | 22 | 23 | 24 | 25 | 26 |
| Behenyl trimethyl ammonium chloride | 2.25 | | | 2.25 | | |
| Isopropyl alcohol | 0.6 | 0.5 | 0.5 | 0.6 | 0.5 | 0.5 |
| Behentrimonium methosulfate | | 1.8 | 1.8 | | 1.8 | 1.8 |
| Cetyl alcohol | 1.9 | 1.1 | 1.1 | 1.9 | 1.1 | 1.1 |
| Stearyl alcohol | 4.6 | 2.8 | 2.8 | 4.6 | 2.8 | 2.8 |
| Preservati ves | 0.9 | 0.9 | 0.9 | 0.9 | 0.9 | 0.9 |
| Aminosilicone ¹ | 2.8 | 1.3 | 0.35 | 2.8 | 1.3 | 0.35 |
| Compound 28 | | | | 0.09 | 0.09 | 0.09 |
| Perfume | 0.5 | 0.5 | 0.5 | 0.5 | 0.5 | 0.5 |
| Deionized Water | q.s. to 100% | q.s. to 100% | q.s. to 100% | q.s. to 100% | q.s. to 100% | q.s. to 100% |

Leave on hair compositions, as shown below in TABLE 24, were prepared using conventional methods.

**TABLE 24**

| | Samples | | | | | | |
|---|---|---|---|---|---|---|---|
| Ingredients | 1 Active wt %) | 2 Active wt % | 3 Active wt % | 4 Active wt % | 5 Active wt % | 6 Active wt % | 7 Active wt % |
| Water | Q.S. | QS | QS | QS | QS | QS | QS |
| Alcohol 100% (Ethanol) | 50 | 50 | 0 | 50 | 60 | 25 | 0 |
| Isoproryl Alcohol | 0 | 0 | 0 | 0 | 0 | 0 | 0 |
| Acrylates/C10-30 alkyl acrylate crosspolymer *1 | 0.35 | 0.5 | 0.2 | 0 | 0 | 0 | 0 |
| Carbomer *2 | 0 | 0 | 0 | 0 | 0 | 0 | 0 |
| Polyacrylamide *3 | 0 | 0 | 0 | 0.5 | 0 | 0 | 0 |
| C13-14 Isoparaffin *3 | 0 | 0 | 0 | 0.5 | 0 | 0 | 0 |
| Laureth 7 *3 | 0 | 0 | 0 | 0.1 | 0 | 0 | 0 |
| Polyacrylate crosspolymer-6 *4 | 0 | 0 | 0 | 0 | 0.5 | 0 | 0 |
| Dehydroxanthan Gum *5 | 0 | 0 | 0 | 0.25 | 0 | 0 | 0 |
| Cetyl Alcohol, Sodium Polyacrylate, Glyceryl Stearate, Polysorbate 80, and Caprylic/Capric Triglycerinde *6 | 0 | 0 | 0 | 0 | 0 | 2.5 | 0 |
| Acrylates/Aminoacrylates/C10-30 Alkyl PEG-20 Itaconate Copolymer *7 | 0 | 0 | 0 | 0 | 0 | 0 | 1.5 |
| Zinc pyrithione *8a | 0.1 | 0.2 | 0.07 | 0.1 | 0.1 | 0.1 | 0.1 |
| Zinc Carbonate *8b | 0 | 0.2 | 0 | 0 | 0 | 0 | 0 |
| PEG/PPG 20/23 Dimethicone 430 *9 | 0 | 1 | 0 | 0 | 0 | 0 | 0 |
| Bis-PEG / PPG-16/16 PEG / PPG 16/16 Dimethicone *10 | 0.7 | 0 | 0 | 1 | 0 | 0 | 0 |
| Polyquaternium-4 *111 | 0 | 0 | 0 | 0 | 0 | 0 | 1 |
| Panthenol | 0.15 | 0.5 | 0 | 0.15 | 0 | 0 | 0 |
| Niacinamide | 2.5 | 0 | 0 | 3 | 0 | 0 | 0 |
| Caffeine | 0.75 | 0 | 0 | 1.25 | 0 | 0 | 0 |
| Glycerin | 0.5 | 5 | 0 | 5 | 0 | 0 | 0 |
| Argania Spinosa Kernel Oil *12 | 0 | 0 | 0 | 0 | 0 | 0 | 0 |
| Propylene Glycol | 0 | 0 | 1 | 0 | 0 | 0 | 0 |
| Menthol | 0 | 0 | 0.5 | 0 | 0 | 0 | 0 |
| Polyvinylpyrrolidone *13 | 0 | 1 | 0 | 0 | 0 | 0 | 0 |
| Polyethylene Low Density Powder *14 | 0 | 0.5 | 0 | 0 | 0 | 0 | 0 |
| Tapicoa Starch Polymethylsilsesuioxane *15 | 0 | 0 | 1 | 0 | 0 | 0 | 0 |
| Benzyl Alcohol | 0 | 0 | 0.5 | 0 | 0 | 0 | 0 |
| Methylisothiazolinone *16 | 0 | 0 | 0.05 | 0 | 0 | 0 | 0 |
| PEG-40 Hydrogenated Castor Oil *17 | 0 | 0 | 0.5 | 0 | 0 | 0 | 0 |
| Tetrahydroxypropyl Ethylenediamine *18 | 0.12 | 0 | 0.14 | 0 | 0 | 0 | 0 |
| Triethanolamine *19 | 0 | 0.1 | 0 | 0 | 0 | 0 | 0 |
| Glycolic Acid *20 | 0 | 0 | 0 | 0 | 0 | 0 | 0.25 |
| Citric Acid | 0 | 0 | 0 | 0.008 | 0.005 | 0.005 | 0.005 |
| Compound 28 | 0.09 | 0.09 | 0.09 | 0.09 | 0.09 | 0.09 | 0.09 |

| | | | | | | | |
|---|---|---|---|---|---|---|---|
| *1 as in Carbopol Ultrez 21 available from Lubrizol (Wickliffe, OH) *2 as in Carbopol Ultrez 30 available from Lubrizol *3 as in Sepigel 305 from Seppic (Puteaux, France) *4 as in SepiMax Zen from Seppic *5 as in Amaze XT from AkzoNovel (Amsterdam, Netherlands) *6 as in Jeesperse CPW-CG-02 from Jeen (Fairfield, New Jersey) *7 as in Structure Plus from Akzo Nobel *8a as in ZPT from Lonza Personal Care (Basel, Switzerland) *8b as in Zinc Carbonate from Brueggemann Chemical (Newtown, PA) *9 as in Silsoft 430 Dimethicone Copolyol from Momentive (Waterford, NY) *10 as in Abil Care 85 from Evonik (Zurich, Switzerland) *11 as in Celquat H-100 from Akzo Nobel *12 as in Lipofructyl Argan LS9779 from BASF (Ludwigshafen, Germany) *13 as in PVP K-30 from ISP Technologies (Wayne, New Jersey) *14 as in Microthene FN 510-00 from Equistar Chemicals (Houston, TX) *15 as in Dry Flo TS from Akzo Nobel *16 as in Neolone 950 from Rohm and Haas (Philadelphia, PA) *17 as in Cremophor RH-40 Surfactant from BASF *18 as in Neutrol Te from BASF *19 as in Trolamine from Dow Chemical (Houston, TX) *20 as in Glypure from DuPont (Wilmington, DE) | | | | | | | |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. Non-therapeutic use of a compound comprising the following structure: 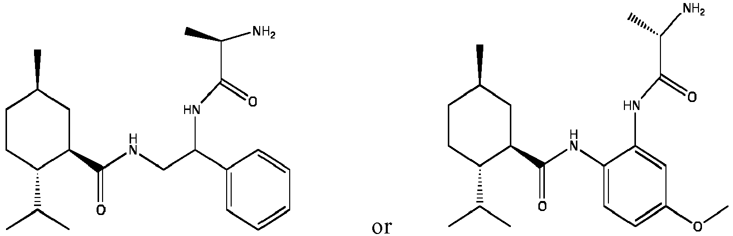 for providing a cooling sensation.

2. A personal care composition comprising a flavor or perfume system comprising one or more coolants of the following structure: 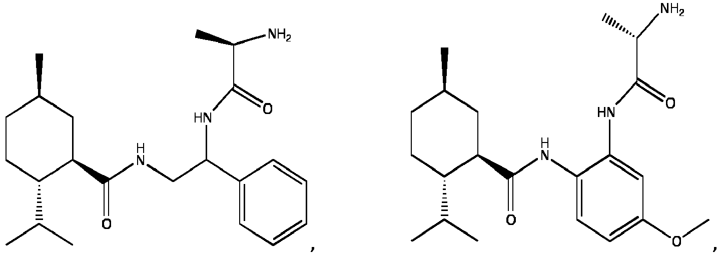 wherein the composition is an oral care composition or a skin care composition .

3. The composition of claim 2, wherein the composition is a dentifrice, floss, chewing gum, or white strip and the level of use of the coolant is from 0.00001% to 0.1%; from 0.00005% to 0.1%; from 0.0001% to 0.05%; or from 0.001% to 0.01% by weight of the composition.

4. The composition of claim 2, wherein the composition is a mouthwash and the level of use of the coolant is from 0.000001% to 0.01% or from 0.0001 % to 0.001 % by weight of the composition.

5. The composition of claim 2, wherein the composition is a shampoo or a lotion and the level of use of the coolant is from 0.001% to 0.5% by weight of the composition or from 0.1% to 0.4% by weight of the composition.

6. The composition of any of claims 2 to 5, wherein the composition further comprises a calcium channel enhancer, preferably phytic acid, polyphosphates with a chain length of greater than or equal to 3, carboxylate polymers, and polyols.

7. The composition of any of claims 2 to 6 comprising further a TRPV1 warming agent, preferably capsaicin, vanillyl butyl ether, vanillyl ethyl ether, zingerone and piperine.

8. The composition of any of claims 2 to 7 and a TRPM8 agonist.

9. The composition of claim 8, wherein the TRPM8 agonists comprises at least one of Menthol; Menthyl Lactate; N-ethyl-p-menthan-3-carboxamide; N-ethoxycarbonylmethyl-ρ-menthan-3-carboxamide; N-(4-methoxyphenyl)-p-menthan-3-carboxamide; N-tert-butyl-ρ-menthan-3-carboxamide; N,2,3-trimethyl-2-isopropylbutanamide; N-(4-cyanomethylphenyl)-p-menthanecarboxamide; N-(4-sulfamoylphenyl)-p-menthanecarboxamide; N-(4-cyanophenyl)-p-menthanecarboxamide,;N-(4-acetylphenyl)-p-menthanecarboxamide; N-(4-hydroxymethylphenyl)-p-menthanecarboxamide; N-(3-hydroxy-4-methoxyphenyl)-p-menthanecarboxamide; Isopulegol; and/or (-)-Menthoxypropane-1,2-diol.

10. The composition of any of claims 2 to 9 and at least one of a TRPA1 agonist or TRPV1 agonist.

11. The composition of claim 10, wherein the TRPA1 agonist is at least one of allyl isothiocyanate; menthol; peroxide; methyl salicylate; cinnamic aldehyde; benzyl alcohol; zinc salts; and/or vanillin isobutyrate, preferably wherein the TRPV1 agonist is at least one of capsaicin; piperine; vanillyl butyl ether; vanillyl ethyl ether; menthol; peroxide; zinc salts; or an anti-histamine.

## Patentansprüche

1. Nichttherapeutische Verwendung einer Verbindung, welche die nachfolgende Struktur umfasst: 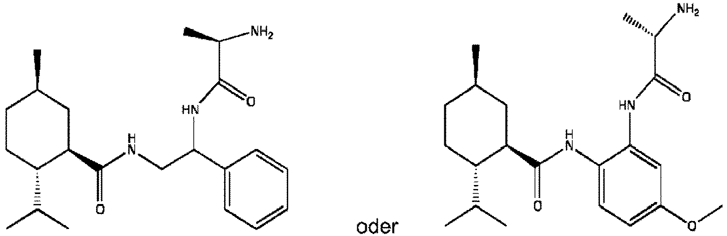 zum Bereitstellen eines kühlenden Gefühls.

2. Körperpflegezusammensetzung, die ein Aroma- oder Duftstoffsystem umfasst, das eines oder mehrere Kühlmittel der folgenden Struktur umfasst: 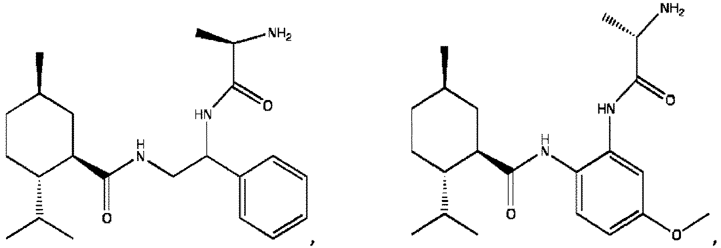 wobei die Zusammensetzung eine Mundpflegezusammensetzung oder eine Hautpflegezusammensetzung ist.

3. Zusammensetzung nach Anspruch 2, wobei die Zusammensetzung eine Zahncreme, Zahnseide, ein Kaugummi, oder ein Aufhellungsstreifen ist und der Anteil der Verwendung des Kühlmittels von 0,00001 Gew.-% bis 0,1 Gew.-%; von 0,00005 Gew.-% bis 0,1 Gew.-%; von 0,0001 Gew.-% bis 0,05 Gew.-%; oder von 0,001 Gew.-% bis 0,01 Gew.-% der Zusammensetzung beträgt.

4. Zusammensetzung nach Anspruch 2, wobei die Zusammensetzung eine Mundspülung ist und der Anteil der Verwendung des Kühlmittels von 0,000001 Gew.-% bis 0,01 Gew.-% oder von 0,0001 Gew.-% bis 0,001 Gew.-% der Zusammensetzung beträgt.

5. Zusammensetzung nach Anspruch 2, wobei die Zusammensetzung ein Shampoo oder eine Lotion ist und der Anteil der Verwendung des Kühlmittels von 0,001 Gew.-% bis 0,5 Gew.-% der Zusammensetzung oder von 0,1 Gew.-% bis 0,4 Gew.-%der Zusammensetzung beträgt.

6. Zusammensetzung nach einem der Ansprüche 2 bis 5, wobei die Zusammensetzung ferner einen Kalziumkanalverstärker, vorzugsweise Phytinsäure, Polyphosphate mit einer Kettenlänge von mehr als oder gleich 3, Carboxylatpolymere und Polyole umfasst.

7. Zusammensetzung nach einem der Ansprüche 2 bis 6, die ferner ein TRPV1 - Erwärmungsmittel umfasst, vorzugsweise Capsaicin, Vanillylbutylether, Vanillylethylether, Zingeron und Piperin.

8. Zusammensetzung nach einem der Ansprüche 2 bis 7 und ein TRPM8-Agonist.

9. Zusammensetzung nach Anspruch 8, wobei die TRPM8-Agonisten mindestens eines umfasst von Menthol; Menthyllactat; N-Ethyl-ρ-menthan-3-carboxamid; N-Ethoxycarbonylmethyl-ρ-menthan-3-carboxamid; N-(4-Methoxyphenyl)-p-menthan-3-carboxamid; N-Tert-butyl-ρ-menthan-3-carboxamid; N,2,3-Trimethyl-2-isopropylbutanamid; N-(4-Cyanomethylphenyl)-ρ-menthancarboxamid; N-(4-Sulfamoylphenyl)-p-menthancarboxamid; N-(4-Cyanophenyl)-p-menthancarboxamid,; N-(4-Acetylphenyl)-ρ-menthancarboxamid; N-(4-Hydroxymethylphenyl)-ρ-menthancarboxamid; N-(3-Hydroxy-4-methoxyphenyl)-ρ-menthancarboxamid; Isopulegol; und/oder (-)-Menthoxypropan-1,2-diol.

10. Zusammensetzung nach einem der Ansprüche 2 bis 9 und mindestens eines von einem TRPA1-Agonisten oder TRPV1-Agonisten.

11. Verfahren nach Anspruch 10, wobei der TRPA1 -Agonist mindestens eines ist von Allylisothiocyanat; Menthol; Peroxid; Methylsalicylat; Zimtaldehyd; Benzylalkohol; Zinksalzen; und/oder Vanillinisobutyrat, wobei vorzugsweise der TRPV1-Agonist mindestens eines ist von Capsaicin; Piperin; Vanillylbutylether; Vanillylethylether; Menthol; Peroxid; Zinksalzen; oder einem Anti-histamin.

## Revendications

1. Utilisation non thérapeutique d'un composé comprenant la structure suivante : 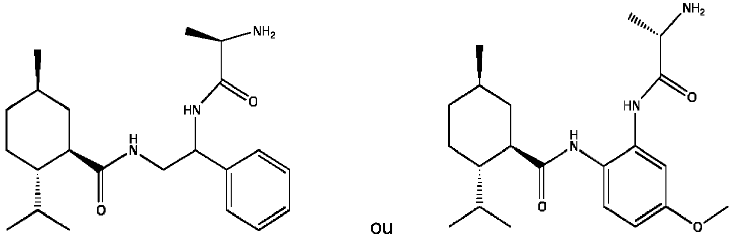 pour fournir une sensation de rafraîchissement.

2. Composition de soins personnels comprenant un système d'arôme ou de parfum comprenant un ou plusieurs agents rafraîchissants de la structure suivante : 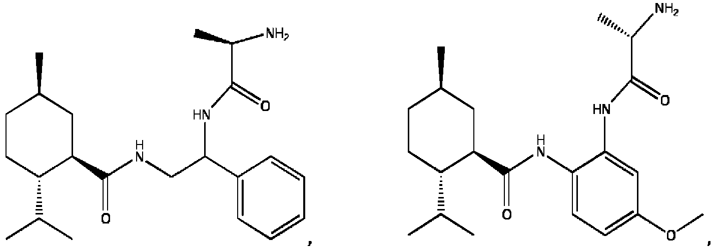 dans laquelle la composition est une composition de soins bucco-dentaires ou une composition de soin de la peau.

3. Composition selon la revendication 2, dans laquelle la composition est un dentifrice, un fil dentaire, un chewing-gum, ou une bandelette de blanchiment et le taux d'utilisation de l'agent rafraîchissant va de 0,00001 % à 0,1 % ; de 0,00005 % à 0,1 % ; de 0,0001 % à 0,05 % ; ou de 0,001 % à 0,01 % en poids de la composition.

4. Composition selon la revendication 2, dans laquelle la composition est un bain de bouche et le taux d'utilisation de l'agent rafraîchissant va de 0,000001 % à 0,01 % ou de 0,0001 % à 0,001 % en poids de la composition.

5. Composition selon la revendication 2, dans laquelle la composition est un shampooing ou une lotion et le taux d'utilisation de l'agent rafraîchissant va de 0,001 % à 0,5 % en poids de la composition ou de 0,1 % à 0,4 % en poids de la composition.

6. Composition selon l'une quelconque des revendications 2 à 5, dans laquelle la composition comprend en outre un activateur de canal calcique, de préférence de l'acide phytique, des polyphosphates avec une longueur de chaîne supérieure ou égale à 3, des polymères carboxylate, et des polyols.

7. Composition selon l'une quelconque des revendications 2 à 6 comprenant en outre un agent réchauffant de TRPV1, de préférence de la capsaïcine, un éther butylique de vanillyle, un éther éthylique de vanillyle, de la zingérone et de la pipérine.

8. Composition selon l'une quelconque des revendications 2 à 7 et un agoniste de TRPM8.

9. Composition selon la revendication 8, dans laquelle les agonistes de TRPM8 comprennent au moins l'un parmi menthol ; lactate de menthyle ; N-éthyl-ρ-menthan-3-carboxamide ; N-éthoxycarbonylméthyl-ρ-menthan-3-carboxamide ; N-(4-méthoxyphényl)-p-menthan-3-carboxamide ; N-tert-butyl-p-menthan-3-carboxamide ; N,2,3-triméthyl-2-isopropylbutanamide ; N-(4-cyanométhylphényl)-ρ-menthane-carboxamide ; N-(4- sulfamoylphényl)-p-menthane-carboxamide ; N-(4-cyanophényl)-p-menthane-carboxamide, ;N-(4-acétylphényl)-ρ-menthane-carboxamide ; N-(4-hydroxyméthylphényl)-ρ-menthane-carboxamide ; N-(3-hydroxy-4-méthoxyphényl)-ρ-menthane-carboxamide ; isopulégol ; et/ou (-)-menthoxypropane-1,2-diol.

10. Composition selon l'une quelconque des revendications 2 à 9 et au moins l'un parmi un agoniste de TRPA1 ou un agoniste de TRPV1.

11. Composition selon la revendication 10, dans laquelle l'agoniste de TRPA1 est au moins l'un parmi isothiocyanate d'allyle ; menthol ; peroxyde ; salicylate de méthyle ; aldéhyde cinnamique ; alcool benzylique ; sels de zinc ; et/ou isobutyrate de vanilline, de préférence dans laquelle l'agoniste de TRPV1 est au moins l'un parmi capsaïcine ; pipérine ; éther butylique de vanillyle ; éther éthylique de vanillyle ; menthol ; peroxyde ; sels de zinc ; ou un antihistaminique.


### SEQUENCE LISTING

<110> The Procter & Gamble Company
<120> COMPOSITIONS FOR DEPOSITION ON BIOLOGICAL SURFACES
<130> 13316M
<160> 3
<170> PatentIn version 3.5
<210> 1
   <211> 2520
   <212> DNA
   <213> Homo sapiens
<400> 1
   atgaagaaat ggagcagcac agacttgggg gcagctgcgg acccactcca aaaggacacc      60
   tgcccagacc ccctggatgg agaccctaac tccaggccac ctccagccaa gccccagctc     120
   tccacggcca agagccgcac ccggctcttt gggaagggtg actcggagga ggctttcccg     180
   gtggattgcc ctcacgagga aggtgagctg gactcctgcc cgaccatcac agtcagccct     240
   gttatcacca tccagaggcc aggagacggc cccaccggtg ccaggctgct gtcccaggac     300
   tctgtcgccg ccagcaccga gaagaccctc aggctctatg atcgcaggag tatctttgaa     360
   gccgttgctc agaataactg ccaggatctg gagagcctgc tgctcttcct gcagaagagc     420
   aagaagcacc tcacagacaa cgagttcaaa gaccctgaga cagggaagac ctgtctgctg     480
   aaagccatgc tcaacctgca cgacggacag aacaccacca tccccctgct cctggagatc     540
   gcgcggcaaa cggacagcct gaaggagctt gtcaacgcca gctacacgga cagctactac     600
   aagggccaga cagcactgca catcgccatc gagagacgca acatggccct ggtgaccctc     660
   ctggtggaga acggagcaga cgtccaggct gcggcccatg gggacttctt taagaaaacc    720
   aaagggcggc ctggattcta cttcggtgaa ctgcccctgt ccctggccgc gtgcaccaac     780
   cagctgggca tcgtgaagtt cctgctgcag aactcctggc agacggccga catcagcgcc     840
   agggactcgg tgggcaacac ggtgctgcac gccctggtgg aggtggccga caacacggcc     900
   gacaacacga agtttgtgac gagcatgtac aatgagattc tgatcctggg ggccaaactg     960
   cacccgacgc tgaagctgga ggagctcacc aacaagaagg gaatgatgcc gctggctctg    1020
   gcagctggga ccgggaagat cggggtcttg gcctatattc tccagcggga gatccaggag    1080
   cccgagtgca ggcacctgtc caggaagttc accgagtggg cctacgggcc cgtgcactcc    1140
   tcgctgtacg acctgtcctg catcgacacc tgcgagaaga actcggtgct ggaggtgatc    1200
   gcctacagca gcagcgagac ccctaatcgc cacgacatgc tcttggtgga gccgctgaac    1260
   cgactcctgc aggacaagtg ggacagattc gtcaagcgca tcttctactt caacttcctg    1320
   gtctactgcc tgtacatgat catcttcacc atggctgcct actacaggcc cgtggatggc    1380
   ttgcctccct ttaagatgga aaaaactgga gactatttcc gagttactgg agagatcctg 1440
   tctgtgttag gaggagtcta cttctttttc cgagggattc agtatttcct gcagaggcgg 1500
   ccgtcgatga agaccctgtt tgtggacagc tacagtgaga tgcttttctt tctgcagtca 1560
   ctgttcatgc tggccaccgt ggtgctgtac ttcagccacc tcaaggagta tgtggcttcc 1620
   atggtattct ccctggcctt gggctggacc aacatgctct actacacccg cggtttccag 1680
   cagatgggca tctatgccgt catgatagag aagatgatcc tgagagacct gtgccgtttc 1740
   atgtttgtct acatcgtctt cttgttcggg ttttccacag cggtggtgac gctgattgaa 1800
   gacgggaaga atgactccct gccgtctgag tccacgtcgc acaggtggcg ggggcctgcc 1860
   tgcaggcccc ccgatagctc ctacaacagc ctgtactcca cctgcctgga gctgttcaag 1920
   ttcaccatcg gcatgggcga cctggagttc actgagaact atgacttcaa ggctgtcttc 1980
   atcatcctgc tgctggccta tgtaattctc acctacatcc tcctgctcaa catgctcatc 2040
   gccctcatgg gtgagactgt caacaagatc gcacaggaga gcaagaacat ctggaagctg 2100
   cagagagcca tcaccatcct ggacacggag aagagcttcc ttaagtgcat gaggaaggcc 2160
   ttccgctcag gcaagctgct gcaggtgggg tacacacctg atggcaagga cgactaccgg 2220
   tggtgcttca gggtggacga ggtgaactgg accacctgga acaccaacgt gggcatcatc 2280
   aacgaagacc cgggcaactg tgagggcgtc aagcgcaccc tgagcttctc cctgcggtca 2340
   agcagagttt caggcagaca ctggaagaac tttgccctgg tccccctttt aagagaggca 2400
   agtgctcgag ataggcagtc tgctcagccc gaggaagttt atctgcgaca gttttcaggg 2460
   tctctgaagc cagaggacgc tgaggtcttc aagagtcctg ccgcttccgg ggagaagtga 2520
<210> 2
   <211> 3360
   <212> DNA
   <213> Homo sapiens
<400> 2
   atgaagtgca gcctgaggaa gatgtggcgc cctggagaaa agaaggagcc ccagggcgtt      60
   gtctatgagg atgtgccgga cgacacggag gatttcaagg aatcgcttaa ggtggttttt     120
   gaaggaagtg catatggatt acaaaacttt aataagcaaa agaaattaaa aacatgtgac     180
   gatatggaca ccttcttctt gcattatgct gcagcagaag gccaaattga gctaatggag     240
   aagatcacca gagattcctc tttggaagtg ctgcatgaaa tggatgatta tggaaatacc     300
   cctctgcatt gtgctgtaga aaaaaaccaa attgaaagcg ttaagtttct tctcagcaga     360
   ggagcaaacc caaacctccg aaacttcaac atgatggctc ctctccacat agctgtgcag     420
   ggcatgaata atgaggtgat gaaggtcttg cttgagcata gaactattga tgttaatttg     480
   gaaggagaaa atggaaacac agctgtgatc attgcgtgca ccacaaataa tagcgaagca     540
   ttgcagattt tgcttaacaa aggagctaag ccatgtaaat caaataaatg gggatgtttc      600
   cctattcacc aagctgcatt ttcaggttcc aaagaatgca tggaaataat actaaggttt      660
   ggtgaagagc atgggtacag tagacagttg cacattaact ttatgaataa tgggaaagcc      720
   acccctctcc acctggctgt gcaaaatggt gacttggaaa tgatcaaaat gtgcctggac      780
   aatggtgcac aaatagaccc agtggagaag ggaaggtgca cagccattca ttttgctgcc      840
   acccagggag ccactgagat tgttaaactg atgatatcgt cctattctgg tagcgtggat      900
   attgttaaca caaccgatgg atgtcatgag accatgcttc acagagcttc attgtttgat      960
   caccatgagc tagcagacta tttaatttca gtgggagcag atattaataa gatcgattct     1020
   gaaggacgct ctccacttat attagcaact gcttctgcat cttggaatat tgtaaatttg     1080
   ctactctcta aaggtgccca agtagacata aaagataatt ttggacgtaa ttttctgcat     1140
   ttaactgtac agcaacctta tggattaaaa aatctgcgac ctgaatttat gcagatgcaa     1200
   cagatcaaag agctggtaat ggatgaagac aacgatgggt gtactcctct acattatgca     1260
   tgtagacagg ggggccctgg ttctgtaaat aacctacttg gctttaatgt gtccattcat     1320
   tccaaaagca aagataagaa atcacctctg cattttgcag ccagttatgg gcgtatcaat     1380
   acctgtcaga ggctcctaca agacataagt gatacgaggc ttctgaatga aggtgacctt     1440
   catggaatga ctcctctcca tctggcagca aagaatggac atgataaagt agttcagctt     1500
   cttctgaaaa aaggtgcatt gtttctcagt gaccacaatg gctggacagc tttgcatcat     1560
   gcgtccatgg gcgggtacac tcagaccatg aaggtcattc ttgatactaa tttgaagtgc     1620
   acagatcgct tggatgaaga cgggaacact gcacttcact ttgctgcaag ggaaggccac     1680
   gccaaagccg ttgcgcttct tctgagccac aatgctgaca tagtcctgaa caagcagcag     1740
   gcctcctttt tgcaccttgc acttcacaat aagaggaagg aggttgttct tacgatcatc     1800
   aggagcaaaa gatgggatga atgtcttaag attttcagtc ataattctcc aggcaataaa     1860
   tgtccaatta cagaaatgat agaatacctc cctgaatgca tgaaggtact tttagatttc     1920
   tgcatgttgc attccacaga agacaagtcc tgccgagact attatatcga gtataatttc     1980
   aaatatcttc aatgtccatt agaattcacc aaaaaaacac ctacacagga tgttatatat     2040
   gaaccgctta cagccctcaa cgcaatggta caaaataacc gcatagagct tctcaatcat     2100
   cctgtgtgta aagaatattt actcatgaaa tggttggctt atggatttag agctcatatg     2160
   atgaatttag gatcttactg tcttggtctc atacctatga ccattctcgt tgtcaatata     2220
   aaaccaggaa tggctttcaa ctcaactggc atcatcaatg aaactagtga tcattcagaa     2280
   atactagata ccacgaattc atatctaata aaaacttgta tgattttagt gtttttatca     2340
   agtatatttg ggtattgcaa agaagcgggg caaattttcc aacagaaaag gaattatttt     2400
   atggatataa gcaatgttct tgaatggatt atctacacga cgggcatcat ttttgtgctg 2460
   cccttgtttg ttgaaatacc agctcatctg cagtggcaat gtggagcaat tgctgtttac 2520
   ttctattgga tgaatttctt attgtatctt caaagatttg aaaattgtgg aatttttatt 2580
   gttatgttgg aggtaatttt gaaaactttg ttgaggtcta cagttgtatt tatcttcctt 2640
   cttctggctt ttggactcag cttttacatc ctcctgaatt tacaggatcc cttcagctct 2700
   ccattgcttt ctataatcca gaccttcagc atgatgctag gagatatcaa ttatcgagag 2760
   tccttcctag aaccatatct gagaaatgaa ttggcacatc cagttctgtc ctttgcacaa 2820
   cttgtttcct tcacaatatt tgtcccaatt gtcctcatga atttacttat tggtttggca 2880
   gttggcgaca ttgctgaggt ccagaaacat gcatcattga agaggatagc tatgcaggtg 2940
   gaacttcata ccagcttaga gaagaagctg ccactttggt ttctacgcaa agtggatcag 3000
   aaatccacca tcgtgtatcc caacaaaccc agatctggtg ggatgttatt ccatatattc 3060
   tgttttttat tttgcactgg ggaaataaga caagaaatac caaatgctga taaatcttta 3120
   gaaatggaaa tattaaagca gaaataccgg ctgaaggatc ttacttttct cctggaaaaa 3180
   cagcatgagc tcattaaact gatcattcag aagatggaga tcatctctga gacagaggat 3240
   gatgatagcc attgttcttt tcaagacagg tttaagaaag agcagatgga acaaaggaat 3300
   agcagatgga atactgtgtt gagagcagtc aaggcaaaaa cacaccatct tgagccttag 3360
<210> 3
   <211> 3315
   <212> DNA
   <213> Homo sapiens
<400> 3
   atgtccttcg agggagccag gctcagcatg aggagccgca gaaatggtac tatgggcagc      60
   acccggaccc tgtactccag tgtatctcgg agcacagacg tgtcctacag tgacagtgat     120
   ttggtgaatt ttattcaggc aaattttaaa aaacgagaat gtgtcttctt taccagagac     180
   tccaaggcca tggagaacat atgcaagtgt ggttatgccc agagccagca catcgaaggc     240
   acccagatca accaaaatga gaagtggaac tacaaaaaac ataccaagga gtttccaaca     300
   gacgccttcg gggacattca gtttgagact ctggggaaga aaggcaagta cttacgcttg      360
   tcctgtgaca ccgactctga aactctctac gaactgctga cccagcactg gcacctcaaa     420
   acacccaacc tggtcatttc agtgacgggt ggagccaaaa actttgcttt gaagccacgc     480
   atgcgcaaga tcttcagcag gctgatttac atcgcacagt ctaaaggtgc gtggattctc     540
   actggaggca ctcactacgg cctgatgaag tacataggcg aggtggtgag agacaacacc     600
   atcagcagga actcagaaga gaacatcgtg gccattggca tcgcagcatg gggcatggtc     660
   tccaacaggg acaccctcat caggagctgt gatgatgagg gacatttttc agctcaatac     720
   atcatggatg actttaccag agaccctcta tacatcctgg acaacaacca tacccacctg 780
   ctgcttgtgg acaacggttg tcatggacac cccacagtgg aagccaagct ccggaatcag 840
   ctggaaaagt acatctctga gcgcaccagt caagattcca actatggtgg taagatcccc 900
   atcgtgtgtt ttgcccaagg aggtggaaga gagactctaa aagccatcaa cacctctgtc 960
   aaaagcaaga tcccttgtgt ggtggtggaa ggctcggggc agattgctga tgtgatcgcc 1020
   agcctggtgg aggtggagga tgttttaacc tcttccatgg tcaaagagaa gctggtacgc 1080
   tttttaccac gcactgtgtc ccggctgcct gaagaggaaa ttgagagctg gatcaaatgg 1140
   ctcaaagaaa ttcttgagag ttctcaccta ctcacagtaa ttaagatgga agaggctgga 1200
   gatgagattg tgagcaacgc catttcctat gcgctgtaca aagccttcag cactaatgag 1260
   caagacaagg acaactggaa tggacagctg aagcttctgc tggagtggaa ccagttggac 1320
   cttgccagtg atgagatctt caccaatgat cgccgctggg agtctgccga ccttcaggag 1380
   gtcatgttca cggctctcat aaaggacaga cccaagtttg tccgcctctt tctggagaat 1440
   ggcctgaatc tgcagaagtt tctcaccaat gaagtcctca cagagctctt ctccacccac 1500
   ttcagcaccc tagtgtaccg gaatctgcag atcgccaaga actcctacaa tgacgcactc 1560
   ctcacctttg tctggaagtt ggtggcaaac ttccgtcgaa gcttctggaa agaggacaga 1620
   agcagcaggg aggacttgga tgtggaactc catgatgcat ctctcaccac ccggcacccg 1680
   ctgcaagctc tcttcatctg ggccattctt cagaacaaga aggaactctc caaggtcatt 1740
   tgggagcaga ccaaaggctg tactctggca gccttggggg ccagcaagct tctgaagacc 1800
   ctggccaaag ttaagaatga tatcaacgct gctggggaat cggaggaact ggccaatgaa 1860
   tatgagaccc gagcagtgga gttgttcacc gagtgttaca gcaatgatga agacttggca 1920
   gaacagctac tggtctactc ctgcgaagcc tggggtggga gcaactgtct ggagctggca 1980
   gtggaggcta cagatcagca tttcatcgct cagcctgggg tccagaattt cctttctaag 2040
   caatggtatg gagagatttc ccgagacacg aagaactgga agattatcct gtgtctattc 2100
   atcatcccct tagtgggctg tggcctcgta tcatttagga agaaacccat tgacaagcac 2160
   aagaagctgc tgtggtacta tgtggccttc ttcacgtcgc ccttcgtggt cttctcctgg 2220
   aacgtggtct tctacatcgc cttcctcctg ctgtttgcct atgtgctgct catggacttc 2280
   cactcagtgc cacacacccc cgagctgatc ctctacgccc tggtcttcgt cctcttctgt 2340
   gatgaagtga ggcagtggta catgaacgga gtgaattatt tcaccgacct atggaacgtt 2400
   atggacaccc tgggactctt ctacttcata gcgggtattg tattccggct ccactcttct 2460
   aataaaagct cgttgtactc tgggcgcgtc attttctgtc tggattacat tatattcacg 2520
   ctaaggctca tccacatttt caccgtcagc aggaacttgg gacccaagat tataatgctg 2580
   cagcggatgc tgatcgacgt tttcttcttc ctgttcctct ttgctgtgtg gatggtggcc 2640
   tttggcgtgg ccagacaggg gatcctaagg caaaatgaac agcgctggag atggatcttc      2700
   cgctctgtca tctatgagcc ctacctggcc atgtttggcc aggttcccag tgacgtggat      2760
   agtaccacat atgacttctc ccactgtacc ttctcgggaa atgagtccaa gccactgtgt      2820
   gtggagctgg atgagcacaa cctgccccgc ttccctgagt ggatcaccat tccgctggtg      2880
   tgcatctaca tgctctccac caatatcctt ctggtcaacc tcctggtcgc catgtttggc      2940
   tacacggtag gcattgtaca ggagaacaac gaccaggtct ggaaattcca gcggtacttc      3000
   ctggtgcagg agtactgcaa ccgcctaaac atccccttcc ccttcgttgt cttcgcttat      3060
   ttctacatgg tggtgaagaa gtgtttcaaa tgctgctgta aagagaagaa tatggagtct      3120
   aatgcctgct gtttcagaaa tgaggacaat gagactttgg cgtgggaggg tgtcatgaag      3180
   gagaattacc ttgtcaagat caacacgaaa gccaacgaca actcagagga gatgaggcat      3240
   cggtttagac aactggactc aaagcttaac gacctcaaaa gtcttctgaa agagattgct      3300
   aataacatca agtaa                                                       3315